(19) Europäisches Patentamt
European Patent Office
Office européen des brevets

(11) EP 4 471 019 A1

(12) EUROPEAN PATENT APPLICATION
published in accordance with Art. 153(4) EPC

(43) Date of publication:
04.12.2024 Bulletin 2024/49

(21) Application number: 23746333.6

(22) Date of filing: 20.01.2023

(51) International Patent Classification (IPC):
*C07D 317/64* (2006.01) *A61K 31/36* (2006.01)
*A61P 25/24* (2006.01)

(52) Cooperative Patent Classification (CPC):
A61K 31/36; A61P 25/24; C07D 317/64

(86) International application number:
PCT/CN2023/073367

(87) International publication number:
WO 2023/143455 (03.08.2023 Gazette 2023/31)

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
KH MA MD TN

(30) Priority: 25.01.2022 CN 202210091412

(71) Applicant: Jiangsu NHWA Pharmaceutical Co., Ltd
Xuzhou, Jiangsu 221000 (CN)

(72) Inventors:
- LI, Quxiang
  Xuzhou, Jiangsu 221000 (CN)
- DUAN, Chen
  Xuzhou, Jiangsu 221000 (CN)
- SONG, Yadong
  Xuzhou, Jiangsu 221000 (CN)
- GUO, Qiang
  Xuzhou, Jiangsu 221000 (CN)
- XU, Xiangqing
  Xuzhou, Jiangsu 221000 (CN)

(74) Representative: Lavoix
Bayerstraße 83
80335 München (DE)

# (54) 3-[(BENZO[D][1,3]DIOXOLANE-4-YL)OXY]PROPYLAMINE HYDROCHLORIDE, OPTICAL ISOMER AND CRYSTAL THEREOF, AND PREPARATION METHOD THEREFOR

(57) The present application relates to a 3-[(benzo[d][1,3]dioxolane-4-yl)oxy]propylamine hydrochloride, a crystalline form thereof, and a preparation method therefor. A crystal form A of an (R)-N-methyl-3-phenyl-3-[(benzo[d][1,3]dioxolane-4-yl)oxy]propylamine hydrochloride prepared in the present invention has good crystal form stability and chemical stability, and can be better used for clinical treatment.

EP 4 471 019 A1

## Description

### TECHNICAL FIELD

The present invention belongs to the field of pharmaceutical chemical engineering, and particularly relates to and provides 3-[(benzo[*d*][1,3]dioxolan-4-yl)oxy]propylamine hydrochloride, and an optical isomer, a crystal form, and a preparation method thereof.

### BACKGROUND

Depression is the most common psychiatric disease endangering human physical and mental health today. Depression patients currently account for 3-5% of the world's population worldwide. It is expected that by 2022 depression will be the 2nd largest disease after heart disease.

Drug therapy is the main means for treating depression. The main therapeutic drugs include: tricyclic antidepressants such as imipramine; monoamine oxidase inhibitors such as moclobemide; selective 5-HT reuptake inhibitors such as fluoxetine; selective NE reuptake inhibitors such as reboxetine; 5-HT and NE dual reuptake inhibitors such as duloxetine.

Although many antidepressants are used clinically, ineffectiveness is still found in a fairly large number of patients after various treatments and some patients still need help with electroconvulsive therapy due to low response rate, long onset time, and potential side effects of some drugs. Therefore, the development of antidepressants is still the hotspot of new drug research. Patent Application WO2016101898A discloses a class of 3-[(benzo[*d*][1,3]dioxolan-4-yl)-oxy]-3-arylpropylamine compounds with antidepressant activity, and the specific structure of the compounds is as follows:

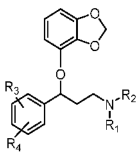

.

Further research finds that (*R*)-*N*-methyl-3-phenyl-3-[(benzo[*d*][1,3]dioxolan-4-yl)oxy]propylamine hydrochloride has good antidepressant activity. The structure of a crystal form of a pharmaceutical active ingredient generally affects the chemical stability of a medicine, and the differences in crystalline forms, preparation methods, and storage conditions may cause changes in the structure of the crystal form of the compound and sometimes generation of other crystalline forms. Generally, amorphous drug products have no regular crystal structure and often have other defects, such as poor product stability, difficulty in filtration, ease of agglomeration, and poor flowability. The differences often cause difficulty in production amplification. Therefore, it is necessary to improve various properties of the compounds through crystalline form and carry out intensive researchto explore novel crystalline forms with high purity and good chemical stability.

### SUMMARY

The technical problem to be solved in the present invention is to provide compounds *N*-methyl-3-phenyl-3-[(benzo[*d*][1,3]dioxolan-4-yl)oxy]propylamine hydrochloride and (*R*)-*N*-methyl-3-phenyl-3-[(benzo[*d*][1,3]dioxolan-4-yl)oxy] propylamine hydrochloride, wherein the (*R*)-*N*-methyl-3-phenyl-3-[(benzo[*d*][1,3]dioxolan-4-yl)oxy]propylamine hydrochloride is a compound of formula (I), and an A crystal form, a B crystal form, and a C crystal form of the compound. The A crystal form described above has good crystal form stability and chemical stability, and can be better applied to clinical applications,

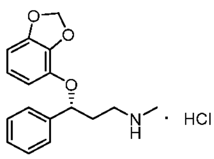

(I)

The present invention provides an A crystal form of (*R*)-*N*-methyl-3-phenyl-3-[(benzo[*d*][1,3]dioxolan-4-yl)oxy] propylamine hydrochloride, and an X-ray powder diffraction pattern at $2\theta \pm 0.2°$ angles of diffraction is obtained using Cu-K$\alpha$ radiation.

Specifically, the A crystal form shows 3 characteristic peaks at 8.77°, 12.00°, and 14.84°. The error range may be ±0.3°, ±0.2°, or ±0.1°.

Specifically, the A crystal form shows 4 characteristic peaks at 8.77°, 12.00°, 14.84°, and 21.34°. The error range may be ±0.3°, ±0.2°, or ±0.1°.

Specifically, the A crystal form shows 5 characteristic peaks at 8.77°, 12.00°, 14.84°, 21.34°, and 21.87°. The error range may be ±0.3°, ±0.2°, or ±0.1°.

Specifically, the A crystal form shows 6 characteristic peaks at 8.77°, 12.00°, 14.84°, 21.34°, 21.87°, and 23.47°. The error range may be ±0.3°, ±0.2°, or ±0.1°.

Specifically, the A crystal form shows 7 characteristic peaks at 8.77°, 12.00°, 14.84°, 21.34°, 21.87°, 23.47°, and 24.14°. The error range may be ±0.3°, ±0.2°, or ±0.1°.

In a preferred embodiment of the present invention, the A crystal form also shows a characteristic peak at 2θ angles at one or more of 7.78°, 16.58°, 19.66°, 23.03°, 25.46°, 26.65°, 26.97°, 27.41°, and 28.23°. The error range may be ±0.3°, ±0.2°, or ±0.1°.

In a preferred embodiment of the present invention, the A crystal form also shows 3 characteristic peaks at 20 angles at 7.78°, 16.58°, and 19.66°. The error range may be ±0.3°, ±0.2°, or ±0.1°.

In a preferred embodiment of the present invention, the A crystal form also shows 4 characteristic peaks at 2θ angles at 7.78°, 16.58°, 19.66°, and 23.03°. The error range may be ±0.3°, ±0.2°, or ±0.1°.

In a preferred embodiment of the present invention, the A crystal form also shows 5 characteristic peaks at 20 angles at 7.78°, 16.58°, 19.66°, 23.03°, and 25.46°. The error range may be ±0.3°, ±0.2°, or ±0.1°.

In a preferred embodiment of the present invention, the A crystal form also shows 6 characteristic peaks at 20 angles at 7.78°, 16.58°, 19.66°, 23.03°, 25.46°, and 26.65°. The error range may be ±0.3°, ±0.2°, or ±0.1°.

In a preferred embodiment of the present invention, the A crystal form also shows 7 characteristic peaks at 20 angles at 7.78°, 16.58°, 19.66°, 23.03°, 25.46°, 26.65°, and 26.97°. The error range may be ±0.3°, ±0.2°, or ±0.1°.

In a preferred embodiment of the present invention, the A crystal form also shows 8 characteristic peaks at 2θ angles at 7.78°, 16.58°, 19.66°, 23.03°, 25.46°, 26.65°, 26.97°, and 27.41°. The error range may be ±0.3°, ±0.2°, or ±0.1°.

In a preferred embodiment of the present invention, the A crystal form also shows 9 characteristic peaks at 2θ angles at 7.78°, 16.58°, 19.66°, 23.03°, 25.46°, 26.65°, 26.97°, 27.41°, and 28.23°. The error range may be ±0.3°, ±0.2°, or ±0.1°. In a more preferred embodiment of the present invention, the Raman spectrum of the A crystal form shows a characteristic peak at one or more of 3063.8±2 cm$^{-1}$, 3013.2±2 cm$^{-1}$, 2961.9±2 cm$^{-1}$, 2931.4±2 cm$^{-1}$, 1599.5±2 cm$^{-1}$, 1461.0±2 cm$^{-1}$, 1267.6±2 cm$^{-1}$, 1177.9±2 cm$^{-1}$, 1047.8±2 cm$^{-1}$, 996.6±2 cm$^{-1}$, 827.9±2 cm$^{-1}$, 740.3±2 cm$^{-1}$, 688.9±2 cm$^{-1}$, 615.4±2 cm$^{-1}$, and 591.9±2 cm$^{-1}$.

In a more preferred embodiment of the present invention, the Raman spectrum of the A crystal form shows 3 characteristic peaks at 3063.8±2 cm$^{-1}$, 3013.2±2 cm$^{-1}$, and 2961.9±2 cm$^{-1}$.

In a more preferred embodiment of the present invention, the Raman spectrum of the A crystal form shows 4 characteristic peaks at 3063.8±2 cm$^{-1}$, 3013.2±2 cm$^{-1}$, 2961.9±2 cm$^{-1}$, and 2931.4±2 cm$^{-1}$.

In a more preferred embodiment of the present invention, the Raman spectrum of the A crystal form shows 5 characteristic peaks at 3063.8±2 cm$^{-1}$, 3013.2±2 cm$^{-1}$, 2961.9±2 cm$^{-1}$, 2931.4±2 cm$^{-1}$, and 1599.5±2 cm$^{-1}$.

In a more preferred embodiment of the present invention, the Raman spectrum of the A crystal form shows 6 characteristic peaks at 3063.8±2 cm$^{-1}$, 3013.2±2 cm$^{-1}$, 2961.9±2 cm$^{-1}$, 2931.4±2 cm$^{-1}$, 1599.5±2 cm$^{-1}$, and 1461.0±2 cm$^{-1}$.

In a more preferred embodiment of the present invention, the Raman spectrum of the A crystal form shows 7 characteristic peaks at 3063.8±2 cm$^{-1}$, 3013.2±2 cm$^{-1}$, 2961.9±2 cm$^{-1}$, 2931.4±2 cm$^{-1}$, 1599.5±2 cm$^{-1}$, 1461.0±2 cm$^{-1}$, and 1267.6±2 cm$^{-1}$.

In a more preferred embodiment of the present invention, the Raman spectrum of the A crystal form shows 8 characteristic peaks at 3063.8±2 cm$^{-1}$, 3013.2±2 cm$^{-1}$, 2961.9±2 cm$^{-1}$, 2931.4±2 cm$^{-1}$, 1599.5±2 cm$^{-1}$, 1461.0±2 cm$^{-1}$, 1267.6±2 cm$^{-1}$, and 1177.9±2 cm$^{-1}$.

In a more preferred embodiment of the present invention, the Raman spectrum of the A crystal form shows 9 characteristic peaks at 3063.8±2 cm$^{-1}$, 3013.2±2 cm$^{-1}$, 2961.9±2 cm$^{-1}$, 2931.4±2 cm$^{-1}$, 1599.5±2 cm$^{-1}$, 1461.0±2 cm$^{-1}$, 1267.6±2 cm$^{-1}$, 1177.9±2 cm$^{-1}$, and 1047.8±2 cm$^{-1}$.

In a more preferred embodiment of the present invention, the Raman spectrum of the A crystal form shows 10 characteristic peaks at 3063.8±2 cm$^{-1}$, 3013.2±2 cm$^{-1}$, 2961.9±2 cm$^{-1}$, 2931.4±2 cm$^{-1}$, 1599.5±2 cm$^{-1}$, 1461.0±2 cm$^{-1}$, 1267.6±2 cm$^{-1}$, 1177.9±2 cm$^{-1}$, 1047.8±2 cm$^{-1}$, and 996.6±2 cm$^{-1}$.

In a more preferred embodiment of the present invention, the Raman spectrum of the A crystal form shows 11 characteristic peaks at 3063.8±2 cm$^{-1}$, 3013.2±2 cm$^{-1}$, 2961.9±2 cm$^{-1}$, 2931.4±2 cm$^{-1}$, 1599.5±2 cm$^{-1}$, 1461.0±2 cm$^{-1}$, 1267.6±2 cm$^{-1}$, 1177.9±2 cm$^{-1}$, 1047.8±2 cm$^{-1}$, 996.6±2 cm$^{-1}$, and 827.9±2 cm$^{-1}$.

In a more preferred embodiment of the present invention, the Raman spectrum of the A crystal form shows 12 characteristic peaks at 3063.8±2 cm$^{-1}$, 3013.2±2 cm$^{-1}$, 2961.9±2 cm$^{-1}$, 2931.4±2 cm$^{-1}$, 1599.5±2 cm$^{-1}$, 1461.0±2 cm$^{-1}$,

1267.6±2 $cm^{-1}$, 1177.9±2 $cm^{-1}$, 1047.8±2 $cm^{-1}$, 996.6±2 $cm^{-1}$, 827.9±2 $cm^{-1}$, and 740.3±2 $cm^{-1}$.

In a more preferred embodiment of the present invention, the Raman spectrum of the A crystal form shows 13 characteristic peaks at 3063.8±2 $cm^{-1}$, 3013.2±2 $cm^{-1}$, 2961.9±2 $cm^{-1}$, 2931.4±2 $cm^{-1}$, 1599.5±2 $cm^{-1}$, 1461.0±2 $cm^{-1}$, 1267.6±2 $cm^{-1}$, 1177.9±2 $cm^{-1}$, 1047.8±2 $cm^{-1}$, 996.6±2 $cm^{-1}$, 827.9±2 $cm^{-1}$, 740.3±2 $cm^{-1}$, and 688.9±2 $cm^{-1}$.

In a more preferred embodiment of the present invention, the Raman spectrum of the A crystal form shows 14 characteristic peaks at 3063.8±2 $cm^{-1}$, 3013.2±2 $cm^{-1}$, 2961.9±2 $cm^{-1}$, 2931.4±2 $cm^{-1}$, 1599.5±2 $cm^{-1}$, 1461.0±2 $cm^{-1}$, 1267.6±2 $cm^{-1}$, 1177.9±2 $cm^{-1}$, 1047.8±2 $cm^{-1}$, 996.6±2 $cm^{-1}$, 827.9±2 $cm^{-1}$, 740.3±2 $cm^{-1}$, 688.9±2 $cm^{-1}$, and 615.4 ±2 $cm^{-1}$.

In a more preferred embodiment of the present invention, the Raman spectrum of the A crystal form shows 15 characteristic peaks at 3063.8±2 $cm^{-1}$, 3013.2±2 $cm^{-1}$, 2961.9±2 $cm^{-1}$, 2931.4±2 $cm^{-1}$, 1599.5±2 $cm^{-1}$, 1461.0±2 $cm^{-1}$, 1267.6±2 $cm^{-1}$, 1177.9±2 $cm^{-1}$, 1047.8±2 $cm^{-1}$, 996.6±2 $cm^{-1}$, 827.9±2 $cm^{-1}$, 740.3±2 $cm^{-1}$, 688.9±2 $cm^{-1}$, 615.4±2 $cm^{-1}$, and 591.9±2 $cm^{-1}$.

In a more preferred embodiment of the present invention, the melting endothermic peak of the DSC of the A crystal form is selected from 161.4 °C to 168.5 °C, preferably 164.8 °C.

Specifically, the melting endothermic peak of the DSC of the A crystal form is selected from 161.4 °C to 164.8 °C.

Specifically, the melting endothermic peak of the DSC of the A crystal form is selected from 161.4 °C to 165.5 °C.

Specifically, the melting endothermic peak of the DSC of the A crystal form is selected from 161.4 °C to 166.5 °C.

Specifically, the melting endothermic peak of the DSC of the A crystal form is selected from 161.4 °C to 167.5 °C.

Specifically, the melting endothermic peak of the DSC of the A crystal form is selected from 164.8 °C to 168.5 °C.

Specifically, the melting endothermic peak of the DSC of the A crystal form is selected from 164.8 °C to 167.5 °C.

Specifically, the melting endothermic peak of the DSC of the A crystal form is selected from 164.8 °C to 166.5 °C.

Specifically, the melting endothermic peak of the DSC of the A crystal form is selected from 164.8 °C to 165.5 °C.

Specifically, the melting endothermic peak of the DSC of the A crystal form may be 161.4 °C, 161.5 °C, 161.6 °C, 161.7 °C, 161.8 °C, 161.9 °C, 162 °C, 162.1 °C, 162.2 °C, 162.3 °C, 162.4 °C, 162.5 °C, 162.6 °C, 162.7 °C, 162.8 °C, 162.9 °C, 163.0 °C, 163.1 °C, 163.2 °C, 163.3 °C, 163.4 °C, 163.5 °C, 163.6 °C, 163.7 °C, 163.8 °C, 163.9 °C, 164.0 °C, 164.1 °C, 164.2 °C, 164.3 °C, 164.4 °C, 164.5 °C, 164.6 °C, 164.7 °C, 164.8 °C, 164.9 °C, 165.0 °C, 165.1 °C, 165.2 °C, 165.3 °C, 165.4 °C, 165.5 °C, 165.6 °C, 165.7 °C, 165.8 °C, 165.9 °C, 166.0 °C, 166.1 °C, 166.2 °C, 166.3 °C, 166.4 °C, 166.5 °C, 166.6 °C, 166.7 °C, 166.8 °C, 166.9 °C, 167.0 °C, 167.1 °C, 167.2 °C, 167.3 °C, 167.4 °C, 167.5 °C, 167.6 °C, 167.7 °C, 167.8 °C, 167.9 °C, 168.0 °C, 168.1 °C, 168.2 °C, 168.3 °C, 168.4 °C, or 168.5 °C.

In a more preferred embodiment of the present invention, the A crystal form is consistent with one or more of the following solid state characteristics:

(I) X-ray powder diffraction pattern substantially consistent with FIG. 1;
(II) DSC pattern substantially consistent with FIG. 2;
(III) Raman spectrum substantially consistent with FIG. 3;

The present invention provides a method for preparing (*R*)-*N*-methyl-3-phenyl-3-[(benzo[*d*][1,3]dioxolan-4-yl) oxy]propylamine hydrochloride, which comprises the following reaction steps:
Acetophenone is used as a starting material, which is subjected to Mannich reaction, reduction, and chiral resolution to give (*R*)-3-(dimethylamino)-1-phenylpropanol mandelate, which is freed, reacted with 3-fluoro-1,2-methylenedioxybenzene, and then subjected to demethylation to give (*R*)-*N*-methyl-3-phenyl-3-[(benzo[*d*][1,3]-dioxolan-4-yl)oxy]propylamine, which is formed into a salt using a HCl-ethyl acetate solution.

The present invention provides a method for preparing an A crystal form of (*R*)-*N*-methyl-3-phenyl-3-[(benzo[*d*][1,3]dioxolan-4-yl)oxy]propylamine hydrochloride, wherein the method is selected from:

**Method I**

1) dissolving (*R*)-*N*-methyl-3-phenyl-3-[(benzo[*d*][1,3]dioxolan-4-yl)oxy]propylamine hydrochloride in a solvent to give a solution containing (*R*)-*N*-methyl-3-phenyl-3-[(benzo[*d*][1,3]dioxolan-4-yl)oxy]propylamine hydrochloride;
2) removing the solvent in the solution prepared in step 1) by a solvent removal method to give a precipitate;

wherein the solvent in step 1) is selected from a good solvent or a mixed solvent of a good solvent and a poor solvent, wherein the good solvent is one or more of alcohols, halogenated hydrocarbons, *N*-methyl-2-pyrrolidone, nitriles, water, *N*,*N*-dimethylformamide, or dimethyl sulfoxide; the alcohol is preferably methanol, ethanol, *n*-propanol, isopropanol, or *n*-butanol, the halogenated hydrocarbon is preferably dichloromethane or chloroform, and the nitrile is selected from acetonitrile; the poor solvent is selected from $C_{5-10}$ saturated hydrocarbons, ketones, esters, or ethers; the $C_{5-10}$ saturated

hydrocarbon is preferably *n*-pentane, *n*-hexane, cyclohexane, or *n*-heptane, the ether is selected from methyl *tert*-butyl ether, isopropyl ether, petroleum ether, or tetrahydrofuran, the ketone is selected from acetone and butanone, and the ester is selected from ethyl acetate, *n*-propyl acetate, isopropyl acetate, or isobutyl acetate.

In a further preferred embodiment of the present invention, the solvent in step 1) is selected from water, chloroform, methanol, ethanol, *N*,*N*-dimethylformamide, dimethylsulfoxide, and *N*-methyl-2-pyrrolidone.

In a further preferred embodiment of the present invention, the solvent in step 1) is a mixed solvent of a $C_{5-10}$ saturated hydrocarbon and any one or more selected from alcohols and halogenated hydrocarbons; a mixed solvent of water and any one or two selected from ketones and alcohols; or a mixed solvent of alcohols and any one or more selected from $C_{5-10}$ saturated hydrocarbons, esters, ethers, and ketones.

In a further preferred embodiment of the present invention, the mixed solvent is selected from methanol/acetonitrile, methanol/ethyl acetate, methanol/water, methanol/methyl *tert*-butyl ether, methanol/isopropyl ether, methanol/-petroleum ether, methanol/*n*-hexane, methanol/*n*-heptane, methanol/cyclohexane, methanol/dichloromethane, ethanol/water, ethanol/acetonitrile, ethanol/ethyl acetate, ethanol/methyl *tert*-butyl ether, ethanol/isopropyl ether, ethanol/petroleum ether, ethanol/*n*-hexane, ethanol/*n*-heptane, ethanol/cyclohexane, ethanol/dichloromethane, 95% ethanol/ethyl acetate, 95% ethanol/acetonitrile, 95% ethanol/*n*-hexane, 95% ethanol/cyclohexane, 95% ethanol/*n*-heptane, 95% ethanol/dichloromethane, *n*-propanol/water, *n*-propanol/acetonitrile, *n*-propanol/EA, *n*-propanol/methyl *tert*-butyl ether, *n*-propanol/isopropyl ether, *n*-propanol/petroleum ether, *n*-propanol/*n*-hexane, *n*-propanol/n-heptane, *n*-propanol/cyclohexane, *n*-propanol/dichloromethane, isopropanol/water, isopropanol/acetonitrile, isopropanol/EA, isopropanol/methyl *tert*-butyl ether, isopropanol/isopropyl ether, isopropanol/petroleum ether, isopropanol/*n*-hexane, isopropanol/*n*-heptane, isopropanol/cyclohexane, isopropanol/dichloromethane, acetonitrile/water, acetonitrile/EA, acetonitrile/methyl *tert*-butyl ether, acetonitrile/isopropyl ether, acetonitrile/dichloromethane, *n*-butanol/water, *n*-butanol/acetonitrile, *n*-butanol/EA, *n*-butanol/methyl *tert*-butyl ether, *n*-butanol/isopropyl ether, *n*-butanol/petroleum ether, *n*-butanol/dichloromethane, acetone/methanol, acetone/ethanol, acetone/95% ethanol, acetone/*n*-propanol, acetone/isopropanol, acetone/*n*-butanol, acetone/acetonitrile, butanone/methanol, butanone/ethanol, butanone/95% ethanol, butanone/*n*-propanol, butanone/*n*-butanol, butanone/isopropanol, chloroform/methanol, chloroform/ethanol, chloroform/*n*-propanol, chloroform/n-butanol, chloroform/isopropanol, chloroform/acetonitrile, chloroform/methyl tert-butyl ether, chloroform/isopropyl ether, chloroform/petroleum ether, chloroform/n-hexane, chloroform/n-heptane, chloroform/cyclohexane, chloroform/acetone, and chloroform/butanone. In a further preferred embodiment of the present invention, the ethanol has a moisture content less than or equal to 5% (v/v).

In other preferred embodiments of the present invention, step 1) further comprises a heating process, wherein the heating temperature of the heating process is selected from a temperature not higher than the boiling point of the solvent used in step 1).

In a preferred embodiment of the present invention, the solvent removal method in step 2) is selected from a reduced pressure solvent removal method, wherein the reduced pressure solvent removal method is preferably a vacuum evaporation method. With the reduced pressure solvent removal method, pressure is gradually reduced from 300 mbar to 10 mbar and the temperature is increased from 20 °C to 90 °C.

In a preferred embodiment of the present invention, the solvent removal method in step 2) is selected from evaporation in a gas stream, wherein the gas stream is preferably an air stream or an inert gas stream, wherein the inert gas is preferably an argon or nitrogen stream.

**Method II**

1) dissolving formula (*R*)-*N*-methyl-3-phenyl-3-[(benzo[*d*][1,3]dioxolan-4-yl)oxy]propylamine hydrochloride in a solvent to give a solution containing (*R*)-*N*-methyl-3-phenyl-3-[(benzo[*d*][1,3]dioxolan-4-yl)oxy]propylamine hydrochloride;
2) obtaining a precipitate from the solution obtained in step 1) by a precipitation method,

wherein the solvent in step 1) is selected from a good solvent or a mixed solvent of a good solvent and a poor solvent, wherein the good solvent is one or more of alcohols, halogenated hydrocarbons, *N*-methyl-2-pyrrolidone, nitriles, water, *N*,*N*-dimethylformamide, or dimethyl sulfoxide; the alcohol is preferably methanol, ethanol, *n*-propanol, isopropanol, or *n*-butanol, the halogenated hydrocarbon is preferably dichloromethane or chloroform, and the nitrile is selected from acetonitrile; the poor solvent is selected from $C_{5-10}$ saturated hydrocarbons, ketones, esters, or ethers; the $C_{5-10}$ saturated hydrocarbon is preferably *n*-pentane, *n*-hexane, cyclohexane, or *n*-heptane, the ether is selected from methyl *tert*-butyl ether, isopropyl ether, petroleum ether, or tetrahydrofuran, the ketone is selected from acetone and butanone, and the ester is selected from ethyl acetate, *n*-propyl acetate, isopropyl acetate, or isobutyl acetate.

In a further preferred embodiment of the present invention, the solvent in step 1) is selected from water,

chloroform, methanol, ethanol, *N*,*N*-dimethylformamide, dimethylsulfoxide, and *N*-methyl-2-pyrrolidone.

In a further preferred embodiment of the present invention, the solvent in step 1) is a mixed solvent of a $C_{5-10}$ saturated hydrocarbon and any one or more selected from alcohols and halogenated hydrocarbons; a mixed solvent of water and any one or two selected from ketones and alcohols; or a mixed solvent of alcohols and any one or more selected from $C_{5-10}$ saturated hydrocarbons, esters, ethers, and ketones.

In a further preferred embodiment of the present invention, the mixed solvent is selected from ethanol/methyl *tert*-butyl ether, ethanol/*n*-hexane, ethanol/*n*-heptane, methanol/methyl *tert*-butyl ether, methanol/isopropyl ether, methanol/ethyl acetate, methanol/acetone, ethanol/acetonitrile, methanol/ethyl acetate, ethanol/ethyl acetate, ethanol/methyl *tert*-butyl ether, acetonitrile/ethyl acetate, acetonitrile/methyl *tert*-butyl ether, acetonitrile/isopropyl ether, acetonitrile/petroleum ether, acetonitrile/*n*-heptane, *n*-propanol/cyclohexane, *n*-propanol/petroleum ether, isopropanol/ethyl acetate, isopropanol/methyl *tert*-butyl ether, chloroform/ethanol, chloroform/n-hexane, chloroform/methyl *tert*-butyl ether, chloroform/acetone, acetonitrile/butanone, and chloroform/acetone.

In a further preferred embodiment of the present invention, the ethanol has a moisture content less than or equal to 5% (v/v).

In a preferred embodiment of the present invention, the precipitation method in step 2) is selected from a cooling method or a precipitant method.

In a preferred embodiment of the present invention, the cooling method in step 2) refers to cooling the solution obtained in step 1) to a specific temperature so that a crystal is precipitated; the specific temperature is selected from 30 °C or lower, preferably room temperature, or preferably 20 °C or lower, preferably 9 °C or lower, and more preferably 0 °C or lower; the error range of the temperature is ±5 °C, preferably ±2 °C.

In another preferred embodiment of the present invention, the cooling method in step 2) refers to cooling the solution obtained in step 1) to a specific temperature so that a crystal is precipitated; the specific temperature is selected from at least 20 °C lower than the temperature of the solution obtained in step 1), preferably at least 30 °C lower than the temperature of the solution obtained in step 1), and more preferably at least 60 °C lower than the temperature of the solution obtained in step 1); the error range of the temperature is ±5 °C, preferably ±2 °C.

In a preferred embodiment of the present invention, the precipitant method in step 2) refers to adding a precipitant of the compound of formula (I) to the solution obtained in step 1) so that a crystal is precipitated; the precipitant is selected from one or more of $C_{5-10}$ saturated hydrocarbons, ketones, esters, or ethers.

In a further preferred embodiment of the present invention, the $C_{5-10}$ saturated hydrocarbon is selected from one or more of *n*-pentane, *n*-hexane, cyclohexane, or *n*-heptane.

In a further preferred embodiment of the present invention, the ether is selected from one or more of methyl *tert*-butyl ether, isopropyl ether, petroleum ether, or tetrahydrofuran, preferably one or more of methyl *tert*-butyl ether or isopropyl ether.

In a further preferred embodiment of the present invention, the ketone is selected from acetone and butanone.

In a further preferred embodiment of the present invention, the ester is selected from ethyl acetate, *n*-propyl acetate, isopropyl acetate, or isobutyl acetate.

In a preferred embodiment of the present invention, step 2) further comprises a precipitation time starting to obtain a precipitate from the solution obtained in step 1) after adding the precipitant. The precipitation time is selected from 0-120 min, 0-60 min, 0-30 min, 0-10 min, 0-5 min, 0-2 min, 0-30 s, or 0 s, preferably 0-10 min, 0-5 min, 0-2 min, 0-30 s, or 0 s, wherein the "0" or "0 s" refers to the time point at which the precipitant is immediately added.

In a further preferred embodiment of the present invention, step 2) further comprises a precipitation time to obtain the maximum precipitation amount selected from 0-90 min, 0-80 min, 0-70 min, or 0-60 min, preferably 0-70 min or 0-60 min, and most preferably 0-60 min, wherein the "0" refers to the time point at which the precipitant is completely added, and the "maximum precipitation amount" refers to the amount that the compound of formula (I) completely precipitates or at least 85% precipitates (mass ratio of the precipitation amount to the dissolved amount of the compound of formula (I)) in a precipitated form from the solution obtained in step 1).

In another preferred embodiment of the present invention, step 2) further comprises a precipitation temperature selected from 0-60 °C, preferably 5-40 °C, and more preferably 15-25 °C; the error range of the temperature is ±5 °C, preferably ±2 °C.

In a preferred embodiment of the present invention, method I or method II further comprises the following steps:

3) separating the solid precipitate obtained in step 2) of method I or method II;
4) drying the solid precipitate obtained in step 3).

In a preferred embodiment of the present invention, step 3) further comprises a separation temperature selected from 0-60 °C, preferably 5-40 °C, and more preferably 15-25 °C; the error range of the temperature is ±5 °C, preferably ±2 °C.

In a preferred embodiment of the present invention, step 4) further comprises a drying temperature selected from

0-60 °C, preferably 5-40 °C, and more preferably 15-25 °C; the error range of the temperature is ±5 °C, preferably ±2 °C.

In a preferred embodiment of the present invention, step 4) further comprises a drying gas stream condition, wherein the drying gas stream is selected from an argon or nitrogen stream.

The present invention provides a B crystal form of (*R*)-*N*-methyl-3-phenyl-3-[(benzo[*d*][1,3]dioxolan-4-yl)oxy] propylamine hydrochloride, and an X-ray powder diffraction pattern at 2θ±0.2° angles of diffraction is obtained using Cu-Kα radiation. The B crystal form shows 3 characteristic peaks at 6.69°, 10.36°, and 11.90°. The error range may be ±0.3°, ±0.2°, or ±0.1°. Specifically, the B crystal form shows 4 characteristic peaks at 6.69°, 10.36°, 11.90°, and 19.69°. The error range may be ±0.3°, ±0.2°, or ±0.1°. Specifically, the B crystal form shows 5 characteristic peaks at 6.69°, 10.36°, 11.90°, 19.69°, and 21.80°. The error range may be ±0.3°, ±0.2°, or ±0.1°. Specifically, the B crystal form shows 6 characteristic peaks at 6.69°, 10.36°, 11.90°, 19.69°, 21.80°, and 24.20°. The error range may be ±0.3°, ±0.2°, or ±0.1°. Specifically, the B crystal form shows 7 characteristic peaks at 6.69°, 10.36°, 11.90°, 19.69°, 21.80°, 24.20°, and 25.63°. The error range may be ±0.3°, ±0.2°, or ±0.1°. Specifically, the B crystal form shows 8 characteristic peaks at 6.69°, 10.36°, 11.90°, 19.69°, 21.80°, 24.20°, 25.63°, and 26.10°. The error range may be ±0.3°, ±0.2°, or ±0.1°. Specifically, the B crystal form shows 9 characteristic peaks at 6.69°, 10.36°, 11.90°, 19.69°, 21.80°, 24.20°, 25.63°, 26.10°, and 28.07°. The error range may be ±0.3°, ±0.2°, or ±0.1°.

In a more preferred embodiment of the present invention, the Raman spectrum of the B crystal form shows 3 characteristic peaks at 3063.6±2 $cm^{-1}$, 3044.5±2 $cm^{-1}$, and 2964.8±2 $cm^{-1}$. Specifically, the Raman spectrum of the B crystal form shows 4 characteristic peaks at 3063.6±2 $cm^{-1}$, 3044.5±2 $cm^{-1}$, 2964.8±2 $cm^{-1}$, and 2918.5±2 $cm^{-1}$. Specifically, the Raman spectrum of the B crystal form shows 5 characteristic peaks at 3063.6±2 $cm^{-1}$, 3044.5±2 $cm^{-1}$, 2964.8±2 $cm^{-1}$, 2918.5±2 $cm^{-1}$, and 1715.2±2 $cm^{-1}$. Specifically, the Raman spectrum of the B crystal form shows 6 characteristic peaks at 3063.6±2 $cm^{-1}$, 3044.5±2 $cm^{-1}$, 2964.8±2 $cm^{-1}$, 2918.5±2 $cm^{-1}$, 1715.2±2 $cm^{-1}$, and 1599.9±2 $cm^{-1}$. Specifically, the Raman spectrum of the B crystal form shows 7 characteristic peaks at 3063.6±2 $cm^{-1}$, 3044.5±2 $cm^{-1}$, 2964.8±2 $cm^{-1}$, 2918.5±2 $cm^{-1}$, 1715.2±2 $cm^{-1}$, 1599.9±2 $cm^{-1}$, and 1458.7±2 $cm^{-1}$. Specifically, the Raman spectrum of the B crystal form shows 8 characteristic peaks at 3063.6±2 $cm^{-1}$, 3044.5±2 $cm^{-1}$, 2964.8±2 $cm^{-1}$, 2918.5±2 $cm^{-1}$, 1715.2±2 $cm^{-1}$, 1599.9±2 $cm^{-1}$, 1458.7±2 $cm^{-1}$, and 1277.4±2 $cm^{-1}$. Specifically, the Raman spectrum of the B crystal form shows 9 characteristic peaks at 3063.6±2 $cm^{-1}$ , 3044.5±2 $cm^{-1}$, 2964.8±2 $cm^{-1}$, 2918.5±2 $cm^{-1}$, 1715.2±2 $cm^{-1}$, 1599.9±2 $cm^{-1}$, 1458.7±2 $cm^{-1}$, 1277.4±2 $cm^{-1}$, and 1177.7±2 $cm^{-1}$. Specifically, the Raman spectrum of the B crystal form shows 10 characteristic peaks at 3063.6±2 $cm^{-1}$, 3044.5±2 $cm^{-1}$, 2964.8±2 $cm^{-1}$, 2918.5±2 $cm^{-1}$, 1715.2±2 $cm^{-1}$, 1599.9±2 $cm^{-1}$, 1458.7±2 $cm^{-1}$, 1277.4±2 $cm^{-1}$, 1177.7±2 $cm^{-1}$, and 1048.2±2 $cm^{-1}$. Specifically, the Raman spectrum of the B crystal form shows 11 characteristic peaks at 3063.6±2 $cm^{-1}$, 3044.5±2 $cm^{-1}$, 2964.8±2 $cm^{-1}$, 2918.5 ±2 $cm^{-1}$, 1715.2±2 $cm^{-1}$, 1599.9±2 $cm^{-1}$, 1458.7±2 $cm^{-1}$, 1277.4±2 $cm^{-1}$, 1177.7±2 $cm^{-1}$, 1048.2±2 $cm^{-1}$, and 996.9±2 $cm^{-1}$. Specifically, the Raman spectrum of the B crystal form shows 12 characteristic peaks at 3063.6±2 $cm^{-1}$, 3044.5±2 $cm^{-1}$, 2964.8±2 $cm^{-1}$, 2918.5±2 $cm^{-1}$, 1715.2±2 $cm^{-1}$, 1599.9±2 $cm^{-1}$, 1458.7±2 $cm^{-1}$, 1277.4±2 $cm^{-1}$, 1177.7±2 $cm^{-1}$, 1048.2±2 $cm^{-1}$, 996.9±2 $cm^{-1}$, and 838.0±2 $cm^{-1}$. Specifically, the Raman spectrum of the B crystal form shows 13 characteristic peaks at 3063.6±2 $cm^{-1}$, 3044.5±2 $cm^{-1}$, 2964.8±2 $cm^{-1}$, 2918.5±2 $cm^{-1}$, 1715.2±2 $cm^{-1}$, 1599.9±2 $cm^{-1}$, 1458.7±2 $cm^{-1}$, 1277.4±2 $cm^{-1}$, 1177.7±2 $cm^{-1}$, 1048.2±2 $cm^{-1}$, 996.9±2 $cm^{-1}$, 838.0±2 $cm^{-1}$, and 739.9±2 $cm^{-1}$. Specifically, the Raman spectrum of the B crystal form shows 14 characteristic peaks at 3063.6±2 $cm^{-1}$, 3044.5±2 $cm^{-1}$, 2964.8±2 $cm^{-1}$, 2918.5±2 $cm^{-1}$, 1715.2±2 $cm^{-1}$, 1599.9±2 $cm^{-1}$, 1458.7±2 $cm^{-1}$, 1277.4±2 $cm^{-1}$, 1177.7±2 $cm^{-1}$, 1048.2±2 $cm^{-1}$, 996.9±2 $cm^{-1}$, 838.0±2 $cm^{-1}$, 739.9±2 $cm^{-1}$, and 691.4±2 $cm^{-1}$. Specifically, the Raman spectrum of the B crystal form shows 15 characteristic peaks at 3063.6±2 $cm^{-1}$, 3044.5±2 $cm^{-1}$, 2964.8±2 $cm^{-1}$, 2918.5±2 $cm^{-1}$, 1715.2 ±2 $cm^{-1}$, 1599.9±2 $cm^{-1}$, 1458.7±2 $cm^{-1}$, 1277.4±2 $cm^{-1}$, 1177.7±2 $cm^{-1}$, 1048.2±2 $cm^{-1}$, 996.9±2 $cm^{-1}$, 838.0±2 $cm^{-1}$, 739.9±2 $cm^{-1}$, 691.4±2 $cm^{-1}$, and 615.4±2 $cm^{-1}$.

In a more preferred embodiment of the present invention, the B crystal form is consistent with one or more of the following solid state characteristics:

(I) X-ray powder diffraction pattern substantially consistent with FIG. 4;
(II) Raman spectrum substantially consistent with FIG. 5;

The present invention provides a method for preparing the B crystal form, wherein the method is selected from a reflux recrystallization method, and the specific method comprises the following steps:

1) dissolving a compound of formula (I) in glacial acetic acid;
2) slowly adding dropwise into methyl *tert*-butyl ether to give a solid precipitate;
3) drying the solid precipitate obtained in step 2).

The present invention provides a C crystal form of (*R*)-*N*-methyl-3-phenyl-3-[(benzo[*d*][1,3]dioxolan-4-yl)oxy] propylamine hydrochloride, and an X-ray powder diffraction pattern at 2θ±0.2° angles of diffraction is obtained using Cu-Kα radiation.

Specifically, the C crystal form shows 3 characteristic peaks at 10.73°, 11.95°, and 14.82°. The error range may be ±0.3°, ±0.2°, or ±0.1°. Specifically, the C crystal form shows 4 characteristic peaks at 10.73°, 11.95°, 14.82°, and 16.14°. The error range may be ±0.3°, ±0.2°, or ±0.1°.

Specifically, the C crystal form shows 5 characteristic peaks at 10.73°, 11.95°, 14.82°, 16.14°, and 16.53°. The error range may be ±0.3°, ±0.2°, or ±0.1°.

Specifically, the C crystal form shows 6 characteristic peaks at 10.73°, 11.95°, 14.82°, 16.14°, 16.53°, and 18.72°. The error range may be ±0.3°, ±0.2°, or ±0.1°. Specifically, the C crystal form shows 7 characteristic peaks at 10.73°, 11.95°, 14.82°, 16.14°, 16.53°, 18.72°, and 18.98°. The error range may be ±0.3°, ±0.2°, or ±0.1°. Specifically, the C crystal form shows 8 characteristic peaks at 10.73°, 11.95°, 14.82°, 16.14°, 16.53°, 18.72°, 18.98°, and 19.52°. The error range may be ±0.3°, ±0.2°, or ±0.1°.

Specifically, the C crystal form shows 9 characteristic peaks at 10.73°, 11.95°, 14.82°, 16.14°, 16.53°, 18.72°, 18.98°, 19.52°, and 21.78°. The error range may be ±0.3°, ±0.2°, or ±0.1°. Specifically, the C crystal form shows 10 characteristic peaks at 10.73°, 11.95°, 14.82°, 16.14°, 16.53°, 18.72°, 18.98°, 19.52°, 21.78°, and 23.00°. The error range may be ±0.3°, ±0.2°, or ±0.1°. Specifically, the C crystal form shows 11 characteristic peaks at 10.73°, 11.95°, 14.82°, 16.14°, 16.53°, 18.72°, 18.98°, 19.52°, 21.78°, 23.00°, and 24.99°. The error range may be ±0.3°, ±0.2°, or ±0.1°. Specifically, the C crystal form shows 12 characteristic peaks at 10.73°, 11.95°, 14.82°, 16.14°, 16.53°, 18.72°, 18.98°, 19.52°, 21.78°, 23.00°, 24.99°, and 26.75°. The error range may be ±0.3°, ±0.2°, or ±0.1°. Specifically, the C crystal form shows 13 characteristic peaks at 10.73°, 11.95°, 14.82°, 16.14°, 16.53°, 18.72°, 18.98°, 19.52°, 21.78°, 23.00°, 24.99°, 26.75°, and 27.25°. The error range may be ±0.3°, ±0.2°, or ±0.1°.

In a more preferred embodiment of the present invention, the C crystal form is consistent with one or more of the following solid state characteristics:

(I) X-ray powder diffraction pattern substantially consistent with FIG. 6;

The present invention provides a method for preparing a C crystal form of a compound of formula (I). Adding a proper amount of water to completely dissolve a sample in water bath at about 65 °C, placing the sample in a refrigerator for pre-freezing, placing the sample in a freeze dryer for freeze-drying experiments after the pre-freezing is completed, and illuminating (5000 lx) for 10 days after the freeze-drying is completed to give the C crystal form.

The present invention further provides a pharmaceutical composition, which comprises any one of active ingredients selected from the A crystal form, the B crystal form, or the C crystal form, and a pharmaceutically acceptable excipient, carrier, adjuvant or vehicle or a combination thereof.

In a preferred embodiment of the present invention, the active ingredient comprises at least 50-99% of the A crystal form, preferably comprises at least 70-99% of the A crystal form, and more preferably comprises at least 90-99% of the A crystal form.

Specifically, the content of the A crystal form in the active ingredient is 50%, 55%, 60%, 65%, 70%, 75%, 80%, 85%, 90%, 95%, or 99%.

In a preferred embodiment of the present invention, the active ingredient comprises at least 50-99% of the B crystal form, preferably comprises at least 70-99% of the B crystal form, and more preferably comprises at least 90-99% of the B crystal form.

Specifically, the content of the B crystal form in the active ingredient is 50%, 55%, 60%, 65%, 70%, 75%, 80%, 85%, 90%, 95%, or 99%.

In a preferred embodiment of the present invention, the active ingredient comprises at least 50-99% of the C crystal form, preferably comprises at least 70-99% of the C crystal form, and more preferably comprises at least 90-99% of the C crystal form.

Specifically, the content of the C crystal form in the active ingredient is 50%, 55%, 60%, 65%, 70%, 75%, 80%, 85%, 90%, 95%, or 99%.

In a further preferred embodiment of the present invention, any one of the A crystal form, the B crystal form, or the C crystal form is present in the active ingredient in substantially pure form. In a preferred embodiment of the present invention, the pharmaceutical composition may be administered by any suitable route, for example orally in the form of capsules, parenterally in the form of injection solutions, topically in the form of ointments or lotions, rectally in the form of suppositories, and transdermally in the form of patch delivery systems. In a preferred embodiment, the pharmaceutical composition is preferably administered orally.

The present invention further relates to use of any one selected from the A crystal form, the B crystal form, or the C crystal form described herein for preparing a pharmaceutical composition, wherein preferably, the pharmaceutical composition is suitable for treating and/or preventing a psychiatric disease. The psychiatric disease is selected from one or more of anxiety, obsessive-compulsive disorder, depression, phobia, and schizophrenia, preferably depression.

The present invention further relates to a method for treating and/or preventing a psychiatric disease, comprising administering to a patient *N*-methyl-3-phenyl-3-[(benzo[*d*][1,3]dioxolan-4-yl)oxy]propylamine hydrochloride or (*R*)-*N*-methyl-3-phenyl-3-[(benzo[*d*][1,3]dioxolan-4-yl)oxy]propylamine hydrochloride or the A crystal form or the aforementioned pharmaceutical composition; the psychiatric disease is selected from one or more of anxiety, obsessive-compulsive disorder, depression, phobia, and schizophrenia, preferably depression.

## Detailed Description of the Invention

In a more preferred embodiment of the present invention, the A crystal form is consistent with one or more of the following solid state characteristics:

(I) X-ray powder diffraction pattern substantially consistent with FIG. 1;
(II) DSC pattern substantially consistent with FIG. 2;
(III) Raman spectrum substantially consistent with FIG. 3;

The present invention provides a method for preparing (*R*)-*N*-methyl-3-phenyl-3-[(benzo[*d*][1,3]dioxolan-4-yl)oxy]propylamine hydrochloride, which comprises the following reaction steps:
Acetophenone is used as a starting material, which is subjected to Mannich reaction, reduction, and chiral resolution to give (*R*)-3-(dimethylamino)-1-phenylpropanol mandelate, which is freed, reacted with 3-fluoro-1,2-methylenedioxybenzene, and then subjected to demethylation to give (*R*)-*N*-methyl-3-phenyl-3-[(benzo[*d*][1,3]-dioxolan-4-yl)oxy]propylamine, which is formed into a salt using a HCl-ethyl acetate solution.
The present invention provides a method for preparing a A crystal form of (*R*)-*N*-methyl-3-phenyl-3-[(benzo[*d*][1,3]dioxolan-4-yl)oxy]propylamine hydrochloride, wherein the method is selected from:

### Method I

1) dissolving (*R*)-*N*-methyl-3-phenyl-3-[(benzo[*d*][1,3]dioxolan-4-yl)oxy]propylamine hydrochloride in a solvent to give a solution containing (*R*)-*N*-methyl-3-phenyl-3-[(benzo[*d*][1,3]dioxolan-4-yl)oxy]propylamine hydrochloride;
2) removing the solvent in the solution prepared in step 1) by a solvent removal method to give a precipitate;

wherein the solvent in step 1) is selected from a good solvent or a mixed solvent of a good solvent and a poor solvent, wherein the good solvent is one or more of alcohols, halogenated hydrocarbons, *N*-methyl-2-pyrrolidone, nitriles, water, *N*,*N*-dimethylformamide, or dimethyl sulfoxide; the alcohol is preferably methanol, ethanol, *n*-propanol, isopropanol, or *n*-butanol, the halogenated hydrocarbon is preferably dichloromethane or chloroform, and the nitrile is selected from acetonitrile; the poor solvent is selected from $C_{5-10}$ saturated hydrocarbons, ketones, esters, or ethers; the $C_{5-10}$ saturated hydrocarbon is preferably *n*-pentane, *n*-hexane, cyclohexane, or *n*-heptane, the ether is selected from methyl *tert*-butyl ether, isopropyl ether, petroleum ether, or tetrahydrofuran, the ketone is selected from acetone and butanone, and the ester is selected from ethyl acetate, *n*-propyl acetate, isopropyl acetate, or isobutyl acetate.
In a more preferred embodiment of the present invention, the alcohol is selected from methanol, ethanol, *n*-propanol, isopropanol, or *n*-butanol; the ester is selected from ethyl acetate, *n*-propyl acetate, isopropyl acetate, or isobutyl acetate; the ketone is selected from acetone, 2-butanone, pentan-2-one, pentan-3-one, hexan-2-one, and hexan-3-one; the ether is selected from methyl *tert*-butyl ether, isopropyl ether, petroleum ether, or tetrahydrofuran; the nitrile is selected from acetonitrile; the halogenated hydrocarbon is selected from dichloromethane or chloroform; the $C_{5-10}$ saturated hydrocarbon is selected from *n*-pentane, *n*-hexane, cyclohexane, or *n*-heptane.
In a further preferred embodiment of the present invention, the solvent in step 1) is selected from water, chloroform, methanol, ethanol, *N*,*N*-dimethylformamide, dimethylsulfoxide, and *N*-methyl-2-pyrrolidone.
In a further preferred embodiment of the present invention, the solvent in step 1) is a mixed solvent of a $C_{5-10}$ saturated hydrocarbon and any one or more selected from alcohols and halogenated hydrocarbons; a mixed solvent of water and any one or two selected from ketones and alcohols; or a mixed solvent of alcohols and any one or more selected from $C_{5-10}$ saturated hydrocarbons, esters, ethers, and ketones.
In a further preferred embodiment of the present invention, the mixed solvent is selected from ethanol/methyl *tert*-butyl ether, ethanol/*n*-heptane, methanol/methyl *tert*-butyl ether, methanol/isopropyl ether, methanol/petroleum ether, methanol/*n*-hexane, methanol/*n*-heptane, methanol/cyclohexane, ethanol/acetonitrile, ethanol/ethyl acetate, ethanol/-methyl *tert*-butyl ether, acetonitrile/ethyl acetate, acetonitrile/methyl *tert*-butyl ether, acetonitrile/isopropyl ether, acetonitrile/petroleum ether, *n*-propanol/dichloromethane (1:2), *n*-propanol/dichloromethane (1:3), methanol/water, isopropanol/ethyl acetate, isopropanol/methyl *tert*-butyl ether, chloroform/ethanol, and chloroform/*n*-hexane.
In a further preferred embodiment of the present invention, the ethanol has a moisture content less than or equal to 5% (v/v).

In other preferred embodiments of the present invention, step 1) further comprises a heating process, wherein the heating temperature of the heating process is selected from a temperature not higher than the boiling point of the solvent used in step 1).

In a preferred embodiment of the present invention, the solvent removal method in step 2) is selected from a reduced pressure solvent removal method, wherein the reduced pressure solvent removal method preferably uses a vacuum solvent removal method. With the reduced pressure solvent removal method, pressure is gradually reduced from 300 mbar to 10 mbar and the temperature is increased from 20 °C to 90 °C.

In a preferred embodiment of the present invention, the solvent removal method in step 2) is selected from evaporation in a gas stream, wherein the gas stream is preferably an air stream or an inert gas stream, wherein the inert gas is preferably an argon or nitrogen stream.

**Method II**

1) dissolving formula (*R*)-*N*-methyl-3-phenyl-3-[(benzo[*d*][1,3]dioxolan-4-yl)oxy]propylamine hydrochloride in a solvent to give a solution containing (*R*)-*N*-methyl-3-phenyl-3-[(benzo[*d*][1,3]dioxolan-4-yl)oxy]propylamine hydrochloride;
2) obtaining a precipitate from the solution obtained in step 1) by a precipitation method,

wherein the solvent in step 1) is selected from a good solvent or a mixed solvent of a good solvent and a poor solvent, wherein the good solvent is one or more of alcohols, halogenated hydrocarbons, *N*-methyl-2-pyrrolidone, nitriles, water, *N*,*N*-dimethylformamide, or dimethyl sulfoxide; the alcohol is preferably methanol, ethanol, *n*-propanol, isopropanol, or *n*-butanol, the halogenated hydrocarbon is preferably dichloromethane or chloroform, and the nitrile is selected from acetonitrile; the poor solvent is selected from $C_{5-10}$ saturated hydrocarbons, ketones, esters, or ethers; the $C_{5-10}$ saturated hydrocarbon is preferably *n*-pentane, *n*-hexane, cyclohexane, or *n*-heptane, the ether is selected from methyl *tert*-butyl ether, isopropyl ether, petroleum ether, or tetrahydrofuran, the ketone is selected from acetone and butanone, and the ester is selected from ethyl acetate, *n*-propyl acetate, isopropyl acetate, or isobutyl acetate.

In a more preferred embodiment of the present invention, the alcohol is selected from methanol, ethanol, *n*-propanol, isopropanol, or *n*-butanol; the ester is selected from ethyl acetate, *n*-propyl acetate, isopropyl acetate, or isobutyl acetate; the ketone is selected from acetone, 2-butanone, pentan-2-one, pentan-3-one, hexan-2-one, and hexan-3-one; the ether is selected from methyl *tert*-butyl ether, isopropyl ether, petroleum ether, or tetrahydrofuran; the nitrile is selected from acetonitrile; the halogenated hydrocarbon is selected from dichloromethane or chloroform; the $C_{5-10}$ saturated hydrocarbon is selected from *n*-pentane, *n*-hexane, cyclohexane, or *n*-heptane.

In a further preferred embodiment of the present invention, the solvent in step 1) is one or more selected from water, chloroform, methanol, ethanol, *N,N*-dimethylformamide, dimethylsulfoxide, and *N*-methyl-2-pyrrolidone.

In a further preferred embodiment of the present invention, the solvent in step 1) is a mixed solvent of a $C_{5-10}$ saturated hydrocarbon and any one or more selected from alcohols and halogenated hydrocarbons; a mixed solvent of water and any one or two selected from ketones and alcohols; or a mixed solvent of alcohols and any one or more selected from $C_{5-10}$ saturated hydrocarbons, esters, ethers, and ketones.

In a further preferred embodiment of the present invention, the mixed solvent is selected from ethanol/methyl *tert*-butyl ether, ethanol/*n*-heptane, methanol/methyl *tert*-butyl ether, methanol/isopropyl ether, methanol/petroleum ether, methanol/*n*-hexane, methanol/*n*-heptane, methanol/cyclohexane, ethanol/acetonitrile, ethanol/ethyl acetate, ethanol/-methyl *tert*-butyl ether, acetonitrile/ethyl acetate, acetonitrile/methyl *tert*-butyl ether, acetonitrile/isopropyl ether, acetonitrile/petroleum ether, *n*-propanol/dichloromethane (1:2), *n*-propanol/dichloromethane (1:3), methanol/water, isopropanol/ethyl acetate, isopropanol/methyl *tert*-butyl ether, chloroform/ethanol, and chloroform/*n*-hexane.

In a further preferred embodiment of the present invention, the ethanol has a moisture content less than or equal to 5% (v/v).

In a preferred embodiment of the present invention, the precipitation method in step 2) is selected from a cooling method or a precipitant method.

In a preferred embodiment of the present invention, the cooling method in step 2) refers to cooling the solution obtained in step 1) to a specific temperature so that a crystal is precipitated; the specific temperature is selected from 30 °C or lower, preferably room temperature, or preferably 20 °C or lower, preferably 9 °C or lower, and more preferably 0 °C or lower; the error range of the temperature is ±5 °C, preferably ±2 °C.

Specifically, the specific temperature may be 30 °C, 29 °C, 28 °C, 27 °C, 26 °C, 25 °C, 24 °C, 23 °C, 22 °C, 21 °C, 20 °C, 19 °C, 18 °C, 17 °C, 16 °C, 15 °C, 14 °C, 13 °C, 12 °C, 11 °C, 10 °C, 9 °C, 8 °C, 7 °C, 6 °C, 5 °C, 4 °C, 3 °C, 2 °C, 1 °C, 0 °C, -1 °C, -2 °C, -3 °C, -4 °C, -5 °C, or the like.

In another preferred embodiment of the present invention, the cooling method in step 2) refers to cooling the

solution obtained in step 1) to a specific temperature so that a crystal is precipitated; the specific temperature is selected from at least 20 °C lower than the temperature of the solution obtained in step 1), preferably at least 30 °C lower than the temperature of the solution obtained in step 1), and more preferably at least 60 °C lower than the temperature of the solution obtained in step 1); the error range of the temperature is ±5 °C, preferably ±2 °C.

Specifically, the specific temperature is selected from at least 25 °C lower than the temperature of the solution obtained in step 1).

Specifically, the specific temperature is selected from at least 35 °C lower than the temperature of the solution obtained in step 1).

Specifically, the specific temperature is selected from at least 40 °C lower than the temperature of the solution obtained in step 1).

Specifically, the specific temperature is selected from at least 45 °C lower than the temperature of the solution obtained in step 1).

Specifically, the specific temperature is selected from at least 50 °C lower than the temperature of the solution obtained in step 1).

Specifically, the specific temperature is selected from at least 55 °C lower than the temperature of the solution obtained in step 1).

Specifically, the specific temperature is selected from at least 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, 50 °C, 51 °C, 52 °C, 53 °C, 54 °C, 55 °C, 56 °C, 57 °C, 58 °C, 59 °C, or 60 °C lower than the temperature of the solution obtained in step 1).

In a preferred embodiment of the present invention, the precipitant method in step 2) refers to adding a precipitant of the compound of formula (I) to the solution obtained in step 1) so that a crystal is precipitated; the precipitant is selected from one or more of $C_{5-10}$ saturated hydrocarbons, ketones, esters, or ethers.

In a further preferred embodiment of the present invention, the $C_{5-10}$ saturated hydrocarbon is selected from one or more of *n*-pentane, *n*-hexane, cyclohexane, or *n*-heptane.

In a further preferred embodiment of the present invention, the ether is selected from one or more of methyl *tert*-butyl ether, isopropyl ether, petroleum ether, or tetrahydrofuran, preferably one or more of methyl *tert*-butyl ether or isopropyl ether.

In a further preferred embodiment of the present invention, the ketone is selected from acetone and butanone.

In a further preferred embodiment of the present invention, the ester is selected from ethyl acetate, *n*-propyl acetate, isopropyl acetate, or isobutyl acetate.

In a preferred embodiment of the present invention, step 2) further comprises a precipitation time starting to obtain a precipitate from the solution obtained in step 1) after adding the precipitant. The precipitation time is selected from 0-120 min, 0-60 min, 0-30 min, 0-10 min, 0-5 min, 0-2 min, 0-30 s, or 0 s, preferably 0-10 min, 0-5 min, 0-2 min, 0-30 s, or 0 s, wherein the "0" or "0 s" refers to the time point at which the precipitant is immediately added.

Specifically, the precipitation time is 0 min, 1 min, 2 min, 3 min, 4 min, 5 min, 6 min, 7 min, 8 min, 9 min, 10 min, 11 min, 12 min, 13 min, 14 min, 15 min, 16 min, 17 min, 18 min, 19 min, 20 min, 21 min, 22 min, 23 min, 24 min, 25 min, 26 min, 27 min, 28 min, 29 min, 30 min, 31 min, 32 min, 33 min, 34 min, 35 min, 36 min, 37 min, 38 min, 39 min, 40 min, 41 min, 42 min, 43 min, 44 min, 45 min, 46 min, 47 min, 48 min, 49 min, 50 min, 51 min, 52 min, 53 min, 54 min, 55 min, 56 min, 57 min, 58 min, 59 min, 60 min, 61 min, 62 min, 63 min, 64 min, 65 min, 66 min, 67 min, 68 min, 69 min, 70 min, 71 min, 72 min, 73 min, 74 min, 75 min, 76 min, 77 min, 78 min, 79 min, 80 min, 81 min, 82 min, 83 min, 84 min, 85 min, 86 min, 87 min, 88 min, 89 min, 90 min, 91 min, 92 min, 93 min, 94 min, 95 min, 96 min, 97 min, 98 min, 99 min, 100 min, 105 min, 110 min, 115 min, or 120 min.

In a further preferred embodiment of the present invention, step 2) further comprises a precipitation time to obtain the maximum precipitation amount selected from 0-90 min, 0-80 min, 0-70 min, or 0-60 min, preferably 0-70 min or 0-60 min, and most preferably 0-60 min, wherein the "0" refers to the time point at which the precipitant is completely added, and the "maximum precipitation amount" refers to the amount that the compound of formula (I) completely precipitates or at least 85% precipitates (mass ratio of the precipitation amount to the dissolved amount of the compound of formula (I)) in a precipitated form from the solution obtained in step 1).

In another preferred embodiment of the present invention, step 2) further comprises a precipitation temperature selected from 0-60 °C, preferably 5-40 °C, and more preferably 15-25 °C; the error range of the temperature is ±5 °C, preferably ±2 °C.

Specifically, the precipitation temperature is 0 °C, 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, 50 °C, 51 °C, 52 °C, 53 °C, 54 °C, 55 °C, 56 °C, 57 °C, 58 °C, 59 °C, or 60 °C.

In a preferred embodiment of the present invention, method I or method II further comprises the following steps:

3) separating the solid precipitate obtained in step 2) of method I or method II;
4) drying the solid precipitate obtained in step 3).

In a preferred embodiment of the present invention, step 3) further comprises a separation temperature selected from 0-60 °C, preferably 5-40 °C, and more preferably 15-25 °C; the error range of the temperature is ±5 °C, preferably ±2 °C.

Specifically, the separation temperature may be 0 °C, 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, 50 °C, 51 °C, 52 °C, 53 °C, 54 °C, 55 °C, 56 °C, 57 °C, 58 °C, 59 °C, or 60 °C.

In a preferred embodiment of the present invention, step 4) further comprises a drying temperature selected from 0-60 °C, preferably 5-40 °C, and more preferably 15-25 °C; the error range of the temperature is ±5 °C, preferably ±2 °C.

Specifically, the drying temperature may be 0 °C, 1 °C, 2 °C, 3 °C, 4 °C, 5 °C, 6 °C, 7 °C, 8 °C, 9 °C, 10 °C, 11 °C, 12 °C, 13 °C, 14 °C, 15 °C, 16 °C, 17 °C, 18 °C, 19 °C, 20 °C, 21 °C, 22 °C, 23 °C, 24 °C, 25 °C, 26 °C, 27 °C, 28 °C, 29 °C, 30 °C, 31 °C, 32 °C, 33 °C, 34 °C, 35 °C, 36 °C, 37 °C, 38 °C, 39 °C, 40 °C, 41 °C, 42 °C, 43 °C, 44 °C, 45 °C, 46 °C, 47 °C, 48 °C, 49 °C, 50 °C, 51 °C, 52 °C, 53 °C, 54 °C, 55 °C, 56 °C, 57 °C, 58 °C, 59 °C, or 60 °C.

In a preferred embodiment of the present invention, step 4) further comprises a drying gas stream condition, wherein the drying gas stream is selected from an argon or nitrogen stream.

In the specification and claims of the present application, unless otherwise indicated, the scientific and technical terms used herein have the meanings commonly understood by those skilled in the art. However, definitions and explanations for some of the related terms are provided below to better understand the present invention. In addition, if the definitions and explanations of the terms provided herein are not consistent with the meanings generally understood by those skilled in the art, the definitions and explanations of the terms provided herein shall prevail.

The "A crystal form" described in the present invention refers to the A crystal form of the compound of formula (I).

The "alcohol" described in the present invention refers to a group derived by substituting one or more hydrogen atoms on "$C_{1-6}$ alkyl" with one or more "hydroxy". The "$C_{1-6}$ alkyl" is a saturated aliphatic hydrocarbon group having 1-6 carbon atoms, including but not limited to methyl, ethyl, propyl, isopropyl, butyl, isobutyl, *n*-butyl, pentyl, and isopentyl. Specific examples of "alcohols" include but are not limited to: methanol, ethanol, *n*-propanol, or isopropanol.

The "ester" described in the present invention refers to a compound having less than 15 carbon atoms, which is formed by the dehydration reaction of an organic acid with an alcohol or phenol, or a lower ester compound having a functional group of -C(O)O- and less than 15 carbon atoms. Specific examples include, but are not limited to: ethyl acetate, dimethyl phthalate, butyl acetate, or propyl acetate.

The "ether" described in the present invention refers to a chain compound or a cyclic compound containing an ether bond -O- and having 1 to 10 carbon atoms. Specific examples include, but are not limited to: diethyl ether, isopropyl ether, propylene glycol methyl ether, tetrahydrofuran, methyl *tert*-butyl ether, or 1,4-dioxane.

The "halogenated hydrocarbon" described in the present invention refers to a group derived by substituting one or more hydrogen atoms on "$C_{1-6}$ alkyl" with one or more "halogen atoms". The "$C_{1-6}$ alkyl" is as defined above. Specific examples include, but are not limited to: methyl chloride, dichloromethane, dichloroethane, chloroform, or carbon tetrachloride.

The "ketone" described in the present invention refers to a compound in which a carbonyl group (-C(O)-) is linked to two hydrocarbon groups, and the ketone can be classified into aliphatic ketone, alicyclic ketone, aromatic ketone, saturated ketone, and unsaturated ketone according to the difference of the hydrocarbon groups in the molecule. Specific examples include, but are not limited to: acetone, butanone (e.g., 2-butanone), pentanone (e.g., pentan-2-one and pentan-3-one), hexanone (e.g., hexan-2-one or hexan-3-one), acetophenone, methyl isobutyl ketone, or methyl pyrrolidone.

The "nitrile" described in the present invention refers to a group derived by substituting one or more hydrogen atoms on "$C_{1-6}$ alkyl" with one or more "cyano". The "$C_{1-6}$ alkyl" is as defined above. The "cyano" is a group in which a carbon atom and a nitrogen atom are connected by a triple bond, and has the chemical formula of -CN. Specific examples include, but are not limited to: acetonitrile or propionitrile.

The "saturated hydrocarbon" described in the present invention refers to a $C_{5-10}$ chain or cyclic alkane, wherein carbon atoms in the molecule are all connected by single bonds, and the remaining bonds are all combined with hydrogen. Specific examples include, but are not limited to: *n*-pentane, *n*-hexane, cyclohexane, or *n*-heptane.

The "mixed solvent" described in the present invention refers to a solvent formed by mixing one or more different solvents according to a certain ratio, wherein the certain ratio is 0.05:1 to 1:0.05, preferably 1:1, 1:2, 1:3, 1:4, 1:5, 1:8, and 1:10.

The "precipitant" described in the present invention refers to "antisolvent" or "anti-solvent", and means that when a component is separated or removed, the component is dissolved in an appropriate solvent in advance, and a solvent insoluble to the component to be separated is added; the precipitant is miscible with a solvent that dissolves the compound

of formula (I).

The good solvent is a solvent having a good solubility for the compound of the present application, and the poor solvent is a solvent having a poor solubility for the compound of the present application or being insoluble for the compound of the present application.

The "boiling point" described in the present invention refers to the boiling point or the azeotropic point of a pure solvent or a mixed solvent.

The "X-ray powder diffraction pattern" or "XRPD" described in the present invention refers to a set of X-ray powder diffraction peaks obtained according to the Bragg's equation 2d $\sin\theta = n\lambda$ (in the equation, $\lambda$ is the wavelength of X-ray, $\lambda$ = 1.54056 Å, the diffraction order n is any positive integer, and the first-order diffraction peak is generally taken, i.e., n = 1). When X-ray is incident on a certain atomic plane with the d-lattice plane spacing of a crystal or a partial crystal sample at a grazing angle $\theta$ (the complementary angle of incidence angle, also known as Bragg angle), the Bragg's equation can be satisfied.

The "20" or "2$\theta$ angle" described in the present invention refers to a diffraction angle. $\theta$ is the Bragg angle in unit ° or degree. The error range of 20 may be $\pm$0.1 to $\pm$0.5, preferably $\pm$0.1 to $\pm$0.3, and more preferably $\pm$0.2.

The "interplanar spacing (d)" described in the present invention refers to 3 selected non-parallel unit vectors (a, b and c), each of which connects two adjacent lattice points and all of which divide the lattice into juxtaposed parallelepiped units. The lattice is divided according to the determined parallelepiped unit connecting lines, giving a set of linear grids called space lattice or crystal lattice. The lattice and the crystal lattice reflect the periodicity of the crystal structure by using geometrical points and lines, respectively. The interplanar spacing (the distance between two adjacent parallel crystal planes) for different crystal planes is different; the unit is Å or Angstrom.

The "differential scanning calorimetry" or "DSC" described in the present invention measures the transition temperature when a crystal absorbs or releases heat due to a change in crystal structure or melting of the crystal. For the same crystal form of the same compound, the thermal transition temperature and melting point errors in continuous analyses may be within about 5 °C, usually within about 3 °C. When a compound is described as having a given DSC peak or melting point, that means the DSC peak or melting point $\pm$5 °C. "Substantially" also takes such temperature variations into account. DSC provides an auxiliary method to identify different crystal forms. Different crystal morphologies can be identified by their different transition temperatures. It should be noted that for a mixture, its DSC peak or melting point may vary over a larger range. Furthermore, the melting temperature is related to the heating rate due to decomposition in the melting process of a substance.

The "Fourier transform Raman spectroscopy" (FT-Raman) used in the present invention is generally used to study the structure and chemical bonds of molecules and can also be used as a method to characterize and identify chemical species. In the present invention, Fourier transform Raman spectroscopy is used to characterize molecular structures and crystal forms. The FT-Raman peak position error range may be $\pm$2 $cm^{-1}$.

The psychiatric diseases in the present invention refer to diseases which are clinically manifested by different degrees of disorders of mental activities such as cognition, emotion, will, and behavior caused by cerebral dysfunction under the influence of various biological, psychological, and social environmental factors. Mental activities include cognitive activities consisting of sensation, perception, attention, memory, thinking and the like, emotional activities, and willing activities. The activity processes are closely related and coordinated with each other to maintain the unified and complete mental activities. The psychiatric disease is mainly divided into mild psychiatric diseases and severe psychiatric diseases. Common mild psychiatric diseases include obsessive-compulsive disorder, depression, and the like. Common severe psychiatric diseases include schizophrenia and the like. The mild psychiatric diseases mainly manifest in emotional disorders such as anxiety and depression and thinking disorders such as obsessive thought, but the thinking of cognition, logical reasoning ability, and self-awareness of patients are basically intact. The early-stage patients with severe psychiatric diseases such as schizophrenia may also have anxiety, obsessive thought behavior, etc., but the cognitive and logical reasoning abilities of the patients are poor, and the self-awareness is almost completely lost. Organic or toxic psychiatric diseases due to brain disorders need to be distinguished from general functional psychiatric diseases. The mild psychiatric diseases include anxiety, obsessive-compulsive disorder, depression, phobia, and the like. Severe psychiatric diseases include schizophrenia and the like. Various psychiatric diseases occur when the body suffers from disorder of brain function caused by internal and external harmful factors. The psychiatric disease is manifested when there is a significant abnormality or disorder in the overall mental activity and the integrity and uniformity of the mental activity is impaired. If the primary issue is a weakening of mental activity capabilities without severe and persistent mental activity disorders, it manifests as neurosis. If the development of mental activities is hindered, it manifests as mental developmental delay.

The depression is mainly characterized by low mood, reduced interest, pessimism, thought retardation, lack of initiative, self-blame and guilt, poor appetite and sleep, worry about various diseases, feeling of discomfort of the whole body and in severe cases, suicidal thoughts and behaviors.

The hydrochloride of the compound of the present invention may comprise one or more asymmetric centers and may therefore exist in various isomeric forms, e.g., as enantiomers and/or diastereomers. For example, the hydrochloride

of the compound described herein may be in the form of individual enantiomers, diastereomers, or geometric isomers, or may be in the form of a mixture of stereoisomers, including racemic mixtures and mixtures enriched in one or more stereoisomers. Isomers may be separated from the mixture by methods known to those skilled in the art, including chiral high performance liquid chromatography (HPLC) and the formation and crystallization of chiral salts; alternatively, preferably, the isomers may be prepared by asymmetric synthesis. See, e.g., Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions, pg. 268 (E. L. Eliel ed., Univ. of Notre Dame Press, Notre Dame, IN 1972). The present invention also encompasses the hydrochloride of the compound described herein as individual isomers substantially free of other isomers and, alternatively, as a mixture of various isomers.

As used herein, a pure enantiomer compound is substantially free of other enantiomers or stereoisomers of the compound (i.e., enantiomeric excess). In other words, the "S" form of the compound is substantially free of the "R" form of the compound and is therefore in enantiomeric excess of the "S" form.

Some of the compounds of the present invention have asymmetric carbon atoms (optical or chiral centers) or double bonds; enantiomers, racemates, diastereomers, tautomers, geometric isomers, and stereoisomeric forms which can be defined according to absolute stereochemistry, such as (R)- or (S)-. The compounds of the present invention do not include compounds known in the art to be too unstable to synthesize and/or isolate. The present invention is intended to include compounds in racemic and optically pure forms. Optically active (R)- and (S)-isomers can be prepared using chiral synthons or chiral reagents, or resolved using conventional techniques.

As used herein, the term "isomer" refers to compounds having the same atomic number and type, and thus the same molecular weight, but differing in the structural arrangement or configuration of the atoms.

As used herein, the term "tautomer" refers to one of two or more structural isomers that exist in equilibrium and are readily converted from one isomeric form into another.

It will be apparent to those skilled in the art that certain compounds of the present invention may exist in tautomeric forms, and all such tautomeric forms of the compounds are within the scope of the present invention.

The term "treating" or "treatment" refers to successfully treating or improving any sign of an injury, disease, lesion, or disorder, including any objective or subjective parameters such as the elimination, alleviation, and mitigation of a symptom, or making the injury, lesion, or disorder more tolerable to the patient; decelerating the rate of degeneration or worsening; making the final outcome of degeneration less debilitating; enhancing the physical or mental health of the patient. Treatment or amelioration of symptoms may be based on objective or subjective parameters, including the results of a physical examination, neuropsychiatric examination, and/or mental assessment.

The term "treating" or "treatment" and related expressions include preventing a lesion, disorder, or disease (e.g., preventing the development of one or more symptoms of the disease, condition, or disorder described herein).

An "effective amount" is an amount sufficient to achieve the stated purpose (e.g., to achieve the effect of administering it, to treat a disease, to reduce enzyme activity, to increase enzyme activity, or to reduce one or more symptoms of a disease or disorder).

An example of an "effective amount" is an amount sufficient to contribute to treatment, prevention, or reduction of one or more symptoms of a disease, which may also be referred to as a "therapeutically effective amount". A "prophylactically effective amount" of a drug is an amount of the drug that, when administered to a subject, will have the intended prophylactic effect, e.g., to prevent or delay the onset (or recurrence) of an injury, disease, lesion, or disorder, or to reduce the likelihood of the onset (or recurrence) of an injury, disease, lesion, or disorder, or a symptom thereof. Complete prevention may not occur with one dose administration and may only occur after a series of doses. Thus, a prophylactically effective amount may be administered through one or more doses. The exact dosage will depend on the therapeutic purpose and can be determined by those skilled in the art using known techniques (see, e.g., Lieberman, Pharmaceutical Dosage Forms (Vol. 1-3, 1992); Lloyd, The Art, Science and Technology of Pharmaceutical Compounding (1999); Pickar, Dosage Calculations (1999); and Remington: The Science and Practice of Pharmacy, 20th edition, 2003, Gennaro ed., Lippincott, Williams & Wilkins).

"Reduction" of one or more symptoms (and grammatical equivalents of the phrase) means to reduce the severity or frequency of the symptoms, or to eliminate the symptoms.

"Control" or "control experiment" is used in its ordinary sense and refers to an experiment in which experimental subjects or reagents are treated as in a parallel experiment, but experimental procedures, reagents, or variables are omitted. In some cases, a control is used as comparative standards to evaluate the efficacy of the experiment.

As used herein, the term "administer", "administration", or "administering" means oral administration, administration in the form of a suppository, surface contact, intravenous, parenteral, intraperitoneal, intramuscular, intralesional, intrathecal, intracranial, intranasal, or subcutaneous administration, or implantation of a slow release device, such as a micro osmotic pump, to a subject. Administration is performed by any route, including parenteral and transmucosal (e.g., buccal, sublingual, palatal, gingival, nasal, vaginal, rectal, or transdermal) administration. Parenteral administration includes, for example, intravenous, intramuscular, intraarterial, intradermal, subcutaneous, intraperitoneal, intraventricular, and intracranial administration. Other modes of delivery include, but are not limited to, the use of a liposome formulation, intravenous infusion, a transdermal patch, and the like.

**Beneficial Effects**

Compared with the prior art, the technical solutions of the present invention have the following advantages: Research shows that the solubility of *N*-methyl-3-phenyl-3-[(benzo[*d*][1,3]dioxolan-4-yl)oxy]propylamine hydrochloride provided by the present invention is better than that of *N*-methyl-3-phenyl-3-[(benzo[*d*][1,3]dioxolan-4-yl)oxy]propylamine oxalate. Compared with *N*-methyl-3-phenyl-3-[(benzo[*d*][1,3]dioxolan-4-yl)oxy]propylamine hydrochloride, the compound of formula (I) has good activity and low toxicity, and is more suitable for medicinal use; the mouse forced swimming pharmacodynamic test shows that the activity of the compound of formula (I) is better than that of *N*-methyl-3-phenyl-3-[(benzo[*d*][1,3]dioxolan-4-yl)oxy]propylamine hydrochloride; the mouse acute toxicity test shows that the toxicity of the compound of formula (I) is lower than that of*N*-methyl-3-phenyl-3-[(benzo[*d*][1,3]dioxolan-4-yl)oxy]propylamine hydrochloride. The A crystal form of the compound of formula (I) of the present invention has high purity and good stability. The hygroscopic weight gain of the A crystal form is 0.01%, with no or almost no hygroscopicity. The purity changes measured by HPLC are small, and the chemical stability is high. The A crystal form of the compound of formula (I) obtained by the technical solution of the present invention can meet the pharmaceutical requirements for production, transportation, and storage. The production process is stable, repeatable, and controllable, which can be suitable for industrial production. In addition, the A crystal form of the present application has good solubility, hygroscopicity, and bioavailability.

**BRIEF DESCRIPTION OF THE DRAWINGS**

FIG. 1 is an X-ray powder diffraction pattern of an A crystal form of (*R*)-*N*-methyl-3-phenyl-3-[(benzo[*d*][1,3]dioxolan-4-yl)oxy]propylamine hydrochloride.

FIG. 2 is a DSC pattern of an A crystal form of (*R*)-*N*-methyl-3-phenyl-3-[(benzo[*d*][1,3]dioxolan-4-yl)oxy]propylamine hydrochloride.

FIG. 3 is a Raman spectrum of an A crystal form of (*R)-N*-methyl-3-phenyl-3-[(benzo[*d*][1,3]dioxolan-4-yl)oxy]propylamine hydrochloride.

FIG. 4 is an X-ray powder diffraction pattern of a B crystal form of (*R*)-*N*-methyl-3-phenyl-3-[(benzo[*d*][1,3]dioxolan-4-yl)oxy]propylamine hydrochloride.

FIG. 5 is a Raman spectrum of a B crystal form of (*R*)-*N*-methyl-3-phenyl-3-[(benzo[*d*][1,3]dioxolan-4-yl)oxy]propylamine hydrochloride.

FIG. 6 is an X-ray powder diffraction pattern of a C crystal form of (*R*)-*N*-methyl-3-phenyl-3-[(benzo[*d*][1,3]dioxolan-4-yl)oxy]propylamine hydrochloride.

FIG. 7 is a DSC pattern of a C crystal form of (*R*)-*N*-methyl-3-phenyl-3-[(benzo[*d*][1,3]dioxolan-4-yl)oxy]propylamine hydrochloride.

**DETAILED DESCRIPTION**

Hereinafter, the present invention will be explained in more detail in reference to examples. The examples are only used to illustrate the technical solutions of the present invention, rather than limit the essence and scope of the present invention.

Test conditions for the instruments used in the experiment:

1. X-ray powder diffraction (XRPD)

Instrument model: Bruker D8 Focus powder X-ray diffractometer.
X-ray parameter: Cu/Kα (λ = 1.540598 Å)
Voltage: 40 kilovolts (kV)
Current: 40 milliamperes (mA)
Scanning range: from 3.0 to 60 degrees
Scanning step length: 0.02 degree
Scanning step speed: 0.5 s/step

2. Differential scanning calorimeter (DSC)

Instrument model: DSC 200F3 differential scanning calorimeter, Netzsch, Germany
Purging gas: nitrogen
Ramping rate: 10.0 K/min
Temperature range: 30-250 °C

3. FT-Raman spectrometer (FT-RM)

Instrument model: Thermo Scientific DXR Smart Raman spectrometer
Diaphragm: 50 μm
Exposure time: 10 s
Exposure frequency: 32 times
Laser: 780 nm
Laser energy: 150 mw

4. High performance liquid chromatograph (HPLC)
Instrument model: Agilent 1260 (DAD) binary pump liquid chromatography
Chromatographic column: Agilent Eclipse XDB (4.6 × 150 mm, 5 μm) C18 column
Mobile phases:
A: 0.01 mol/L KH2PO4 (pH 3.0)-methanol (90:10)
B: methanol-water (90:10)

Flow rate: 1.0 mL/min Column temperature: 35 °C
Wavelength: 210 nm Injection volume: 5 μL

Gradient conditions volume ratio):

| Time (min) | A (%) | B (%) |
|---|---|---|
| 0 | 80 | 20 |
| 60 | 20 | 80 |
| 61 | 80 | 20 |

5. Reagents used in the experiment:

Dichloromethane (analytically pure), ethanol (analytically pure), *n*-heptane (analytically pure), methanol (analytically pure), ethyl acetate (analytically pure), 95% ethanol (analytically pure), methyl *tert*-butyl ether (analytically pure), *n*-hexane (analytically pure), and isopropyl ether (analytically pure) were all purchased from Shanghai Lingfeng Reagent.
3-[(Benzo[*d*][1,3]dioxolan-4-yl)-oxy]-*N*-methyl-3-phenylpropylamine oxalate was prepared with reference to Example 4 in CN105777706A.

Examples:

Example 1:

1.1. Preparation of (*R*)-*N*-methyl-3-phenyl-3-[(benzo[*d*][1,3]dioxolan-4-yl)oxy]propylamine hydrochloride (i.e., compound of formula I)

1.1.1. Preparation of 3-(dimethylamino)propiophenone hydrochloride

100 g (0.832 mol) of acetophenone, 37.5 g (1.24 mol) of paraformaldehyde, 95.0 g (1.16 mol) of dimethylamine hydrochloride, and 300 mL of ethanol were added into a 1 L glass reaction flask. The mixture was mechanically stirred and heated to 50-60 °C. 5 mL of concentrated hydrochloric acid was added, and the temperature was controlled at 80-90 °C to react for 15 h. After the reaction was completed, the reaction solution was cooled for crystallization, cooled to 5-15 °C, stirred for 1 h, and filtered. The filter cake was washed 2 times with anhydrous ethanol. The resulting solid was dried in an air blast dryer to give a white solid (140.84 g).

1.1.2. Preparation of 3-(dimethylamino)-1-phenylpropanol

100 g (0.47 mol) of 3-(dimethylamino)propiophenone hydrochloride and 250 mL of methanol were added into a 1 L reaction flask. The mixture was mechanically stirred. An aqueous sodium hydroxide solution (18.7 g of sodium hydroxide and 100 mL of water) was added. The temperature was controlled at 0-20 °C. 10.66 g (0.28 mol) of sodium borohydride was added in portions. After the addition was completed, the temperature was controlled at 15-25 °C to react for 2 h.
After the reaction was completed, the mixture was filtered, the filter cake was washed with 100 mL of methanol, and the waste residue was discarded. The filtrate was concentrated under reduced pressure to remove methanol, and then extracted with 200 mL of water and 250 mL of ethyl acetate. The aqueous phase was then extracted with ethyl acetate 2 times (150 mL/time). The organic phases were combined, washed 2 times with a saturated sodium chloride solution (200 mL/time), dried over anhydrous magnesium sulfate for 1 h, filtered, and then concentrated under reduced pressure to remove ethyl acetate to give a colorless oil (80.54 g).

1.1.3. Preparation of (*R*)-3-(dimethylamino)-1-phenylpropanol mandelate

80 g (0.45 mol) of 3-(dimethylamino)-1-phenylpropanol, 300 mL of toluene, and 100 mL of anhydrous ethanol were added into a 1 L reaction flask. The mixture was mechanically stirred. The temperature was controlled at 50-60 °C. 54.72 g of R-(-)- mandelic acid was added. The mixture was heated to 90 °C and reacted for 1 h.
After the reaction was completed, the mixture was cooled for crystallization. The temperature was controlled at 10-20 °C, and the mixture was stirred for 1 h. The mixture was filtered. The filter cake was washed with 250 mL of anhydrous ethanol, and the filtrate was discarded. The resulting solid was dried in an air blast dryer to give a white solid (62.63 g). 62.63 g of the white solid described above was added into a 500 mL reaction flask, and 100 mL of toluene and 250 mL of anhydrous ethanol were added for recrystallization to give a white solid (56.1 g).

1.1.4. Preparation of (*R*)-*N*,*N*-dimethyl-3-phenyl-3-[(benzo[d][1,3]-dioxolan-4-yl)oxy]propylamine oxalate

First, (*R*)-3-(dimethylamino)-1-phenylpropanol mandelate was freed to give a base (28.3 g). 28.0 g (0.16 mol) of (*R*)-3-(dimethylamino)-1-phenylpropanol and dimethyl sulfoxide (250 mL) were added into a 500 mL reaction flask. The mixture was mechanically stirred. 23.3 g (0.21 mol) of potassium *tert*-butoxide was added in portions, and then 33.6 g (0.24 mol) of 3-fluoro-1,2-methylenedioxybenzene was added. The mixture was heated to 50-55 °C and reacted for 4 h. After the reaction was completed, the mixture was cooled to 15-30 °C and extracted with 200 mL of water and 250 mL of ethyl acetate. The aqueous phase was then extracted 1 time with 200 mL of ethyl acetate. The organic phases were combined, washed 2 times with a saturated sodium chloride solution, dried over anhydrous magnesium sulfate, and filtered. The filter cake was rinsed with ethyl acetate, and then formed into a salt by oxalic acid to give an off-white solid (36.14 g). Then anhydrous ethanol was added for recrystallization to give a white solid (27.87 g).

1.1.5. Preparation of phenyl (*R*)-*N*-methyl-3-phenyl-3-[(benzo[*d*][1,3]-dioxolan-4-yl)oxy]propylamine carboxylate

First, (*R*)-*N*,*N*-dimethyl-3-phenyl-3-[(benzo[*d*][1,3]-dioxolan-4-yl)oxy]propylamine oxalate was freed to give a base (20.68 g). 20.0 g (0.067 mol) of the base was taken and added into a 500 mL reaction flask. Then 2.59 g (0.02 mol) of *N,N*-diisopropylethylamine and 200 mL of toluene were added. The mixture was stirred magnetically. 12.5 g (0.08 mol) of phenyl chloroformate was added in portions. After the addition was completed, the mixture was heated to 80 °C and reacted for 2 h.
After the reaction was completed, the mixture was cooled to 15-30 °C and extracted and washed with 200 mL of water. The lower aqueous phase was discarded, and the mixture was washed 2 times with 1% hydrochloric acid, and then washed 2 times with a saturated sodium chloride solution. The organic phase was concentrated under reduced pressure to remove toluene to give a tan liquid (26.99 g).

1.1.6. Preparation of (*R*)-*N*-methyl-3-phenyl-3-[(benzo[*d*][1,3]-dioxolan-4-yl)oxy]propylamine hydrochloride

26 g (0.064 mol) of phenyl (*R*)-*N*-methyl-3-phenyl-3-[(benzo[*d*][1,3]-dioxolan-4-yl)oxy]propylamine carboxylate and 250 mL of dimethyl sulfoxide were added into a 500 mL reaction flask. The mixture was stirred magnetically. A sodium hydroxide solution (mixing 20 g of sodium hydroxide and 50 mL of water for dissolution and clarification) was added, and the mixture was heated to 85 °C and reacted for 12 h.
After the reaction was completed, the mixture was cooled to 15-30 °C and extracted with 250 mL of water and 200 mL of ethyl acetate. The aqueous phase was then extracted 2 times with ethyl acetate (200 mL/time). The organic phases were combined, washed 2 times with a saturated sodium chloride solution, dried over anhydrous sodium sulfate, and filtered. The filter cake was rinsed with ethyl acetate, and then concentrated under reduced pressure to remove the solvent

to give a base (14.03 g). Then 150 mL of ethyl acetate was added for dissolution. The mixture was stirred magnetically. A HCl-ethyl acetate solution was slowly added. The pH was adjusted to 3-5. A solid was gradually precipitated. The mixture was stirred for another 1 h and filtered. The filter cake was rinsed with ethyl acetate. The resulting solid was dried in an air blast dryer to give an off-white solid (12.5 g, HPLC: 99.54%). m.p = 162-164 °C; $[\alpha]_D^{20}$ = 48.0; $^1$H-NMR (400MHz, DMSO-$d_6$) δ: 7.40-7.26(m, 4H),7.28 (t, *J* = 6.7 Hz, 1H), 6.64(t, *J* = 8.1 Hz, 1H), 6.50(d, *J* =7.6 Hz, 2H), 5.96(d, *J* =11.0 Hz, 2H), 5.54-5.51(m, 1H), 3.08-2.95(m, 2H), 2.55(s, 3H), 2.32-2.28(m, 1H), 2.25-2.10(m, 1H); $^{13}$C-NMR (100MHz, DMSO-$d_6$) δ:148.89, 141.71, 140.64, 136.15, 129.14, 128.60, 125.48, 122.31, 111.47, 103.12, 101.39, 78.20, 45.78, 34.35, 32.97; HRMS (ESI) m/z: calcd for: $C_{17}H_{19}NO_3$ $(M+H)^+$: 286.1438; found: 286.1432.

1.2. Preparation of *N*-methyl-3-phenyl-3-[(benzo[*d*][1,3]dioxolan-4-yl)oxy]propylamine hydrochloride

1.2.1. Preparation of 3-(dimethylamino)propiophenone hydrochloride

See 1.1.1 in Example 1 for the preparation method.

1.2.2. Preparation of 3-(dimethylamino)-1-phenylpropanol

See 1.1.2 in Example 1 for the preparation method.

1.2.3. Preparation of *N*,*N*-dimethyl-3-phenyl-3-[(benzo[*d*][1,3]-dioxolan-4-yl)oxy]propylamine oxalate

See 1.1.4 in Example 1 for the preparation method. 3-(Dimethylamino)-1-phenylpropanol was used for replacing (*R*)-3-(dimethylamino)-1-phenylpropanol mandelate in the reaction.

1.2.4. Preparation of phenyl *N*-methyl-3-phenyl-3-[(benzo[*d*][1,3]-dioxolan-4-yl)oxy]propylamine carboxylate

See 1.1.5 in Example 1 for the preparation method.

1.2.5. Preparation of *N*-methyl-3-phenyl-3-[(benzo[*d*][1,3]-dioxolan-4-yl)oxy]propylamine hydrochloride

See 1.1.6 in Example 1 for the preparation method. An off-white solid (20.1 g, HPLC: 99.22%) was obtained. $^1$H-NMR (400MHz, DMSO-$d_6$) δ: 7.47-7.33 (m, 4H), 7.30 (d, *J* = 6.5 Hz, 1H), 6.65 (t, *J* = 8.1 Hz, 1H), 6.51 (d, *J* = 15.2, 8.1 Hz, 2H), 5.99 (d, *J* = 11.4 Hz, 2H), 5.52 (dd, *J* = 7.7, 4.4 Hz, 1H), 3.12-2.90 (m, 2H), 2.54 (s, 3H), 2.26 (d, *J* = 8.7 Hz, 1H), 2.19-2.04 (m, 1H); $^{13}$C-NMR (100MHz, DMSO-$d_6$) *δ*: 164.39, 148.94, 141.77, 140.78, 136.15, 129.11, 128.51, 125.46, 122.24, 111.40, 103.06, 101.39, 78.16, 45.64, 34.37, 32.94; HRMS (ESI) m/z: calcd for: $C_{17}H_{19}NO_3$ $(M+H)^+$: 286.1438; found: 286.1435.

Example 2: Preparation of an A crystal form of (*R*)-*N*-methyl-3-phenyl-3-[(benzo[*d*][1,3]dioxolan-4-yl)oxy]propylamine hydrochloride:

Standing and recrystallizing: About 500 mg of (*R*)-*N*-methyl-3-phenyl-3-[(benzo[*d*][1,3]dioxolan-4-yl)oxy]propylamine hydrochloride was weighed and placed into a beaker. 20 mL of anhydrous ethanol was gradually added. The mixture was heated at 60-65 °C to help dissolution and was left to stand at room temperature (about 25 °C) for crystallization. The precipitate was collected, filtered, and dried at 60 °C. The X-ray powder diffraction is shown in FIG. 1, the DSC pattern is shown in FIG. 2, and the Raman spectrum is shown in FIG. 3. In the DSC heating process, the initial point of an endothermic peak was 161.4 °C, the end point was 168.5 °C, and the peak value was 164.8 °C. This crystal form was defined as the A crystal form, and its characteristic peak positions are shown in Table 1 below:

Table 1

| 2θ Angle/ degree | Strength (%) | 2θ Angle/ degree | Strength (%) | 2θ Angle/ degree | Strength (%) | 2θ Angle/ degree | Strength (%) |
|---|---|---|---|---|---|---|---|
| 7.78 | 5.4 | 21.87 | 37.6 | 28.23 | 21.2 | 36.57 | 6.0 |
| 8.77 | 30.0 | 22.47 | 16.0 | 28.78 | 5.1 | 38.61 | 5.7 |
| 12.00 | 100.0 | 23.03 | 33.3 | 29.23 | 8.2 | 39.02 | 6.3 |
| 14.25 | 12.0 | 23.47 | 40.3 | 29.96 | 12.9 | 39.88 | 5.3 |

(continued)

| 2θ Angle/ degree | Strength (%) | 2θ Angle/ degree | Strength (%) | 2θ Angle/ degree | Strength (%) | 2θ Angle/ degree | Strength (%) |
|---|---|---|---|---|---|---|---|
| 14.84 | 33.3 | 24.14 | 59.1 | 30.35 | 14.4 | 44.04 | 6.3 |
| 16.58 | 29.6 | 25.46 | 15.7 | 30.79 | 6.3 | 44.17 | 6.6 |
| 16.95 | 11.6 | 26.04 | 5.8 | 31.49 | 11.0 | 44.58 | 8.9 |
| 17.55 | 6.4 | 26.65 | 16.2 | 32.55 | 8.7 | 48.91 | 5.8 |
| 18.96 | 11.8 | 26.97 | 16.8 | 33.85 | 6.6 | | |
| 19.66 | 28.0 | 27.41 | 29.1 | 34.35 | 7.0 | | |
| 21.34 | 40.1 | 28.06 | 12.2 | 34.57 | 6.0 | | |

Example 3: Preparation of the A crystal form of (*R*)-*N*-methyl-3-phenyl-3-[(benzo[*d*][1,3]dioxolan-4-yl)oxy]propylamine hydrochloride:

About 500 mg of (*R*)-*N*-methyl-3-phenyl-3-[(benzo[*d*][1,3]dioxolan-4-yl)oxy]propylamine hydrochloride was weighed and placed into a beaker. 12 mL of anhydrous methanol was gradually added. The mixture was heated at 60-65 °C to help dissolution. After complete dissolution, 120 mL of methyl *tert*-butyl ether was rapidly added. A solid was precipitated. The precipitate was collected, filtered, and dried at 60 °C, which was detected as the A crystal form.

Example 4: Preparation of the A crystal form of (*R*)-*N*-methyl-3-phenyl-3-[(benzo[*d*][1,3]dioxolan-4-yl)oxy]propylamine hydrochloride:

About 500 mg of (*R*)-*N*-methyl-3-phenyl-3-[(benzo[*d*][1,3]dioxolan-4-yl)oxy]propylamine hydrochloride was weighed and placed into a large test tube. 30 mL of a mixed solvent of dichloromethane and ethanol (volume ratio 1:1) was gradually added. The mixture was heated at 60-65 °C to help dissolution, and transferred to a Buchi parallel reaction instrument. With the solvent removal method, pressure was gradually reduced from 700 mba to 10 mba, and the temperature was increased from 30 °C to 70 °C. After the sample was completely precipitated, it was collected and detected as the A crystal form.

Example 5: Preparation of the A crystal form of (*R*)-*N*-methyl-3-phenyl-3-[(benzo[*d*][1,3]dioxolan-4-yl)oxy]propylamine hydrochloride:

About 500 mg of (*R*)-*N*-methyl-3-phenyl-3-[(benzo[*d*][1,3]dioxolan-4-yl)oxy]propylamine hydrochloride was weighed and placed into a large test tube. A mixed solvent of methanol/*n*-hexane (volume ratio 5:1) was gradually added. The mixture was heated at 60-65 °C to help dissolution, and transferred to a Buchi parallel reaction instrument. With the solvent removal method, pressure was gradually reduced from 700 mba to 10 mba, and the temperature was increased from 30 °C to 70 °C. After the sample was completely precipitated, it was collected and detected as the A crystal form.

Example 6: Preparation of the A crystal form of (*R*)-*N*-methyl-3-phenyl-3-[(benzo[*d*][1,3]dioxolan-4-yl)oxy]propylamine hydrochloride:

About 500 mg of (*R*)-*N*-methyl-3-phenyl-3-[(benzo[*d*][1,3]dioxolan-4-yl)oxy]propylamine hydrochloride was weighed and placed into a small beaker. A mixed solvent of ethanol/isopropyl ether (volume ratio 7:1) was gradually added. The mixture was heated at 60-65 °C to help dissolution. After complete dissolution, the mixture was left to stand at room temperature for crystallization and filtered under reduced pressure. The filter cake was washed 2 times with petroleum ether, recovered, and dried at 60 °C to give a target product of the A crystal form.

Example 7: Preparation of the A crystal form of (*R*)-*N*-methyl-3-phenyl-3-[(benzo[*d*][1,3]dioxolan-4-yl)oxy]propylamine hydrochloride:

About 500 mg of (*R*)-*N*-methyl-3-phenyl-3-[(benzo[*d*][1,3]dioxolan-4-yl)oxy]propylamine hydrochloride was weighed and placed into a small beaker. 80 mL of a mixed solvent of *n*-propanol/acetone (volume ratio 3:1) was gradually added. The mixture was heated at 60-65 °C to help dissolution. After complete dissolution, the mixture was left to stand at room temperature for crystallization and filtered under reduced pressure. The filter cake was washed 2 times with

petroleum ether, recovered, and dried at 60 °C to give a target product of the A crystal form.

Example 8: Preparation of the A crystal form of (*R*)-*N*-methyl-3-phenyl-3-[(benzo[*d*][1,3]dioxolan-4-yl)oxy]propylamine hydrochloride:

About 500 mg of (*R*)-*N*-methyl-3-phenyl-3-[(benzo[*d*][1,3]dioxolan-4-yl)oxy]propylamine hydrochloride was weighed and placed into a small beaker. 100 mL of a mixed solvent of acetonitrile/butanone (volume ratio 5:1) was gradually added. The mixture was heated at 60-65 °C to help dissolution. After complete dissolution, the mixture was left to stand at room temperature for crystallization and filtered under reduced pressure. The filter cake was washed 2 times with petroleum ether, recovered, and dried at 60 °C to give a target product of the A crystal form.

Example 9: Preparation of the A crystal form of (*R*)-*N*-methyl-3-phenyl-3-[(benzo[*d*][1,3]dioxolan-4-yl)oxy]propylamine hydrochloride:

500 mg of (*R*)-*N*-methyl-3-phenyl-3-[(benzo[*d*][1,3]dioxolan-4-yl)oxy]propylamine hydrochloride was dissolved in 20 mL of dimethyl sulfoxide. 200 mL of methyl *tert*-butyl ether was rapidly poured into the solution described above, and a large amount of off-white precipitates were precipitated to form a suspension. The suspension described above was left to stand and filtered under reduced pressure. The filter cake was washed 2 times with petroleum ether, recovered, and dried at 60 °C to give a target product of the A crystal form.

Example 10: Preparation of the A crystal form of (*R*)-*N*-methyl-3-phenyl-3-[(benzo[*d*][1,3]dioxolan-4-yl)oxy]propylamine hydrochloride:

500 mg of (*R*)-*N*-methyl-3-phenyl-3-[(benzo[*d*][1,3]dioxolan-4-yl)oxy]propylamine hydrochloride was dissolved in 20 mL of *N*,*N*-dimethylformamide. 200 mL of isopropyl ether was rapidly poured into the solution described above, and a large amount of off-white precipitates were precipitated to form a suspension. The suspension described above was left to stand and filtered under reduced pressure. The filter cake was washed 2 times with petroleum ether, recovered, and dried at 60 °C to give a target product of the A crystal form.

Example 11: Preparation of the A crystal form of (*R*)-*N*-methyl-3-phenyl-3-[(benzo[*d*][1,3]dioxolan-4-yl)oxy]propylamine hydrochloride:

500 mg of (*R*)-*N*-methyl-3-phenyl-3-[(benzo[*d*][1,3]dioxolan-4-yl)oxy]propylamine hydrochloride was dissolved in 20 mL of *N*,*N*-dimethylformamide. 200 mL of isopropyl ether was rapidly poured into the solution described above, and a large amount of off-white precipitates were precipitated to form a suspension. The suspension described above was left to stand and filtered under reduced pressure. The filter cake was washed 2 times with petroleum ether, recovered, and dried at 60 °C to give a target product of the A crystal form.

Example 12: Preparation of a B crystal form of (*R*)-*N*-methyl-3-phenyl-3-[(benzo[*d*][1,3]dioxolan-4-yl)oxy]propylamine hydrochloride

About 2 g of (*R*)-*N*-methyl-3-phenyl-3-[(benzo[*d*][1,3]dioxolan-4-yl)oxy]propylamine hydrochloride was weighed and placed into a beaker. 11 mL of glacial acetic acid was added, the mixture was heated at about 65 °C to help dissolution for complete dissolution. The mixture was sucked out by a syringe and slowly added dropwise into a container containing a large amount of methyl *tert*-butyl ether after passing through a filter membrane. A solid was precipitated. The mixture was filtered under vacuum and dried at 60 °C. The X-ray powder diffraction is shown in FIG. 4, and the Raman spectrum is shown in FIG. 5. This crystal form was defined as the B crystal form, and its characteristic peak positions are shown in Table 2 below:

Table 2

| 2θ Angle/degree | Strength (%) | 2θ Angle/degree | Strength (%) | 2θ Angle/degree | Strength (%) | 2θ Angle/degree | Strength (%) |
|---|---|---|---|---|---|---|---|
| 3.90 | 2.0 | 15.40 | 5.1 | 19.70 | 23.8 | 24.20 | 13.1 |
| 6.69 | 27.6 | 16.10 | 5.0 | 20.30 | 9.5 | 24.70 | 5.9 |
| 8.74 | 5.1 | 16.64 | 10.9 | 21.04 | 12.4 | 25.63 | 30.2 |
| 10.36 | 100.0 | 17.10 | 8.1 | 21.81 | 15.8 | 26.10 | 42.6 |

(continued)

| 2θ Angle/ degree | Strength (%) | 2θ Angle/ degree | Strength (%) | 2θ Angle/ degree | Strength (%) | 2θ Angle/ degree | Strength (%) |
|---|---|---|---|---|---|---|---|
| 11.90 | 41.3 | 17.78 | 16.2 | 22.59 | 9.1 | 27.05 | 14.2 |
| 14.00 | 11.8 | 18.30 | 5.9 | 23.01 | 10.0 | 28.07 | 24.4 |
| 14.88 | 13.5 | 18.90 | 10.2 | 23.76 | 12.9 | 28.90 | 5.8 |

Example 13: Preparation of a C crystal form of (*R*)-*N*-methyl-3-phenyl-3-[(benzo[*d*][1,3]dioxolan-4-yl)oxy]propylamine hydrochloride

A sample of 5 g (*R*)-*N*-methyl-3-phenyl-3-[(benzo[*d*][1,3]-dioxolan-4-yl)oxy]propylamine hydrochloride was weighed. A proper amount of water was added to completely dissolve the sample in water bath at about 65 °C. The sample was placed in a refrigerator for pre-freezing, placed in a freeze dryer for freeze-drying experiments after the pre-freezing was completed, and illuminated (5000 lx) for 10 days after the freeze-drying was completed to give the C crystal form. The X-ray powder diffraction is shown in FIG. 6, and the DSC pattern is shown in FIG. 7. In the DSC heating process, the initial point of an endothermic peak was 161.2 °C, the end point was 166.0 °C, and the peak value was 163.9 °C. This crystal form was defined as the C crystal form, and its characteristic peak positions are shown in Table 3 below:

Table 3

| 2θ Angle/ degree | Strength (%) | 2θ Angle/ degree | Strength (%) | 2θ Angle/ degree | Strength (%) | 2θ Angle/ degree | Strength (%) |
|---|---|---|---|---|---|---|---|
| 10.73 | 39.9 | 16.94 | 26.8 | 21.31 | 36.0 | 27.25 | 49.1 |
| 11.95 | 59.7 | 18.11 | 36.5 | 21.78 | 74.7 | 28.21 | 22.6 |
| 14.24 | 27.3 | 18.72 | 93.5 | 23.00 | 50.5 | 28.77 | 21.1 |
| 14.82 | 42.3 | 18.98 | 52.6 | 24.08 | 25.4 | 29.91 | 20.1 |
| 16.14 | 46.5 | 19.51 | 100.0 | 24.99 | 44.1 | 30.34 | 18.0 |
| 16.53 | 44.3 | 20.48 | 29.3 | 26.75 | 49.5 | 33.19 | 21.2 |

**Example 14: Experiments of influencing factors of A crystal form of compound (I)**

Experimental procedures

High temperature experiment: About 1 g of the A, B, and C crystal forms were respectively weighed, placed in a clean watch glass, and placed at 60 °C for 10 days. The samples were taken on day 0, day 5, day 10, and day 30 for XRPD analysis.

High humidity experiment: About 1 g of the A, B, and C crystal form samples were respectively weighed, placed in a clean watch glass, and placed at a relative humidity of 92.5% for 10 days. The samples were taken on day 0, day 5, day 10, and day 30 for XRPD analysis.

Illumination experiment: About 1 g of the A, B, and C crystal form samples were respectively weighed, placed in a clean watch glass, laid flat, and placed in a photostability tester (the illumination was 5000 lx) for 10 days. The samples were taken on day 0, day 5, day 10, and day 30 for XRPD analysis.

Experimental results

The research results of the stability of the crystal forms A, B, and C of the compound of formula (I) under high temperature, high humidity, and illumination are shown in Table 4, Table 5, and Table 6.

Table 4. Experiments of influencing factors of A crystal form

| Days | High temperature of 60 °C | High humidity of 92.5% | Illumination of 5000 lx |
|---|---|---|---|
| 5 days | Crystal form A | Crystal form A | Crystal form A |
| 10 days | Crystal form A | Crystal form A | Crystal form A |

(continued)

| Days | High temperature of 60 °C | High humidity of 92.5% | Illumination of 5000 lx |
|---|---|---|---|
| 30 days | Crystal form A | Crystal form A | Crystal form A |

Table 5. Experiments of influencing factors of B crystal form

| Days | High temperature of 60 °C | High humidity of 92.5% | Illumination of 5000 lx |
|---|---|---|---|
| 5 days | Crystal form A + B | Crystal form A + B | Crystal form B |
| 10 days | Crystal form A + B | Crystal form A + B | Crystal form B |
| 30 days | Crystal form A + B | Crystal form A + B | Crystal form B |

Table 6. Experiments of influencing factors of C crystal form

| Days | High temperature of 60 °C | High humidity of 92.5% | Illumination of 5000 lx |
|---|---|---|---|
| 5 days | Crystal form C | Crystal form A | Crystal form C |
| 10 days | Crystal form C | Crystal form A | Crystal form C |
| 30 days | Crystal form C | Crystal form A | Crystal form C |

Experimental conclusion: The A crystal form can keep stable under high temperature, high humidity, and illumination, the crystal form is not changed, while the B crystal form is converted into a mixed crystal of the crystal forms A and B under the high temperature and high humidity environment, and the C crystal form is converted into the A crystal form under the high humidity environment.

**Example 15: Pharmacokinetic assay**

Pharmacokinetic assays of the A crystal form of (*R*)-*N*-methyl-3-phenyl-3-[(benzo[*d*][1,3]dioxolan-4-yl)oxy] propylamine hydrochloride (compound of formula I) and *N*-methyl-3-phenyl-3-[(benzo[*d*][1,3]dioxolan-4-yl)oxy]propylamine oxalate.

Table 7. Administration regimen:

| | *N*-methyl-3-phenyl-3-[(benzo[*d*][1,3] dioxolan-4-yl)oxy]propylamine oxalate | | Compound of formula I | | Crystal form A of compound of formula (I) | |
|---|---|---|---|---|---|---|
| | Intravenous | Oral | Intravenous | Oral | Intravenous | Oral |
| Dose (mg/kg) | 0.3 | 1 | 0.3 | 1 | 0.3 | 1 |

Each group had six beagle dogs, half male and half female. The beagle dogs were purchased from Shanghai Jiao Tong University School of Agriculture.

Preparation of solutions:

1) Intravenous injection administration: 0.9% Normal saline was used as a vehicle.
2) Oral administration: According to the dose of 10 mg/kg (based on base), a corresponding mass of the drug was weighed according to the actual weight of the animal, and then filled into a capsule to be administered to the animal. The test results are shown in Table 8.

Table 8

| Group | *N*-methyl-3-phenyl-3-[(benzo [*d*][1,3]dioxolan-4-yl)oxy] propylamine oxalate | | Compound of formula I | | Crystal form A of compound of formula (I) | |
|---|---|---|---|---|---|---|
| Administration route | Intravenous | Oral | Intravenous | Oral | Intravenous | Oral |

(continued)

| Group | *N*-methyl-3-phenyl-3-[(benzo [*d*][1,3]dioxolan-4-yl)oxy] propylamine oxalate | | Compound of formula I | | Crystal form A of compound of formula (I) | |
|---|---|---|---|---|---|---|
| Dose (mg/kg) | 0.3 | 1 | 0.3 | 1 | 0.3 | 1 |
| Pharmacokinetic parameters | Mean | Mean | Mean | Mean | Mean | Mean |
| Cmax (ng/mL) | 71.6 | 23.7 | 80.4 | 28.9 | 83.6 | 35.3 |
| $T_{max}$(h) | 0.08 | 1.43 | 0.08 | 1.23 | 0.08 | 0.92 |
| $t_{1/2}$ (h) | 1.93 | 1.98 | 2.15 | 2.36 | 2.08 | 2.48 |
| AUC(0-t) (ug/L*h) | 109.3 | 78.7 | 117.4 | 100.3 | 123.7 | 116.3 |
| AUC(0-∞) (ug/L*h) | 110.2 | 79.9 | 118.8 | 101.5 | 124.5 | 117.1 |
| Bioavailability (%) | / | 21.8% | / | 25.7% | / | 28.2% |

Experimental conclusion: The pharmacokinetic experiments show that the bioavailability of the A crystal form of the compound of formula (I) is better than that of the hydrochloride, and the bioavailability value of the compound of formula (I) is better than that of *N*-methyl-3-phenyl-3-[(benzo[*d*][1,3]dioxolan-4-yl)oxy]propylamine oxalate.

Example 16: Solubility experiment

Solubility experiments of the A crystal form of (*R*)-*N*-methyl-3-phenyl-3-[(benzo[*d*][1,3]dioxolan-4-yl)oxy]propylamine hydrochloride (compound of formula I) and *N*-methyl-3-phenyl-3-[(benzo[*d*][1,3]dioxolan-4-yl)oxy]propylamine oxalate.

16.1 Preparation of samples (the aqueous medium was degassed purified water)

Test sample solution: About 2 g of each sample ((*R*)-*N*-methyl-3-phenyl-3-[(benzo[*d*][1,3]dioxolan-4-yl)oxy] propylamine oxalate, *N*-methyl-3-phenyl-3-[(benzo[*d*][1,3]dioxolan-4-yl)oxy]propylamine hydrochloride, the compound of formula I and the A crystal form thereof, and *N*-methyl-3-phenyl-3-[(benzo[*d*][1,3]dioxolan-4-yl)oxy]propylamine oxalate) was precisely weighed and placed in different volumetric flasks, 2 mL of water was precisely measured, which was used as a concentrated solution of the test sample. The concentrated solution was placed in a 20 °C water bath thermostatic oscillator for overnight oscillation (supersaturation) and filtered. The filtrate was taken, 1 mL of which was precisely measured and placed in a 100 mL volumetric flask. A diluent (water) was added, the mixture was diluted to volume and mixed well, 1 mL of which was precisely measured and placed in a 10 mL volumetric flask. A diluent was added, the mixture was diluted to volume and mixed well as a test sample solution.

Control substance solution: A proper amount of 5 samples were respectively taken, precisely measured, dissolved in a proper amount of a diluent by ultrasonication, and diluted to give a solution containing about 0.1 mg of the sample per 1 mL. Two duplicate samples were prepared for each batch.

16.2. Preparation of mobile phase

(1) Preparation of buffer: About 2.6428 g ammonium sulfate was weighed and dissolved in 1000 mL of water. 2 mL of triethylamine was added, and the pH value was adjusted to 4.0 with dilute sulfuric acid.

(2) Preparation of mobile phase: Methanol-0.02 mol/L ammonium sulfate solution (2 mL triethylamine was added per 1000 mL, and the pH value was adjusted to 4.0 with dilute sulfuric acid) (45:55). 1000 mL was prepared, mixed well, filtered, and used after ultrasonic treatment.

16.3. Chromatographic conditions

Detection wavelength: 230 nm; flow rate: 1.0 mL/min; column temperature: 35 °C; sample injection volume: 20 μL; chromatographic column: Agilent Eclipse XDB-C18 chromatographic column (4.6 × 150 mm, 5 μm); mobile phase: methanol-0.02 mol/L ammonium sulfate solution (2 mL triethylamine was added per 1000 mL, and the pH value was adjusted to 4.0 with dilute sulfuric acid) (45:55).

16.4. Determination of samples

20 μL of each solution was precisely measured, and injected separately into a liquid chromatograph. The chromatograms were recorded, and the solubility of the compounds was determined according to an external standard method. The results are shown in Table 9.

Table 9

| Group | *N*-methyl-3-phenyl-3-[(benzo[*d*][1,3]dioxo lan-4-yl) oxy]propylamine oxalate | (*R*)-*N*-methyl-3-phenyl-3-[(benzo[*d*][1,3]dioxol an-4-yl) oxy]propylamine oxalate | *N*-methyl-3-phenyl-3-[(benzo[*d*][1,3]diox olan-4-yl) oxy]propylamine hydrochloride | Compoun d of formula I | Crystal form A of compound of formula (I) |
|---|---|---|---|---|---|
| Temperature | 20 °C | | | | |
| Medium | Water | | | | |
| Solubility mg/mL | 6.90 | 5.1 | 1500 | 33.24 | 35.55 |

Experimental conclusion: The solubility experiments show that *N*-methyl-3-phenyl-3-[(benzo[*d*][1,3]dioxolan-4-yl)oxy]propylamine hydrochloride has better solubility than the compound of formula (I), the A crystal form of the compound of formula (I) has better solubility than the compound of formula (I), and the compound of formula (I) has better solubility than *N*-methyl-3-phenyl-3-[(benzo[*d*][1,3]dioxolan-4-yl)oxy]propylamine oxalate.

**Example 17: Hygroscopicity test:**

17.1. A dried glass weighing bottle with a plug (outer diameter: 50 mm; height: 15 mm) was taken and placed in a suitable thermostatic dryer (with saturated ammonium chloride or ammonium sulfate solution placed in the lower part) at 25 °C±1 °C or an artificial climate box (with a set temperature at 25 °C±1 °C and relative humidity at 80%±2%) on the day before the test, and the weight ($m_1$) was precisely measured.

17.2. An appropriate amount of the A crystal form of the compound of formula (I) was taken and spread in the above weighing bottle, with the thickness of the test sample being about 1 mm in general, and the weight ($m_2$) was precisely measured.

17.3. The weighing bottle was opened and placed in the above conditions at constant temperature and humidity for 24 h together with the cap.

17.4. The weighing bottle was capped, and the weight ($m_3$) was precisely measured. The test results are shown in Table 10.

$$\text{Weight gain\%} = \frac{m_3 - m_2}{m_2 - m_1} \times 100\%$$

Table 10

| Name of medicine | $m_1$ (g) | $m_2$ (g) | $m_3$ (g) | Weight gain (%) | Hygroscopicity |
|---|---|---|---|---|---|
| Crystal form A of compound of formula (I) | 20.6917 | 21.6890 | 21.6891 | 0.01 | Non-hygroscopic or hardly hygroscopic |

Experimental conclusion: The A crystal form of the compound of formula (I) shows no or almost no hygroscopicity and does not require stringent packaging requirements during storage.

**Example 18: Mouse forced swimming test:**

18.1. Test method

SPF grade ICR mice were taken. The animals were grouped by weight, 12 animals per group, and the animals

were allowed to acclimate for at least 3 days before behavioural test.

The behavioural test was started 1 h after intragastric administration. The mice were placed in a transparent glass cylinder with water (water depth: 15 cm, water temperature: 23-25 °C) and video recorded for 6 min (the background was an LED light panel). At the end of the time, the mice were removed from the water and the next batch of mice was subjected to the behavioural test. Water was changed after every 3 batches of tests. After the test, the video of the behavioural test was derived and the cumulative immobility time of the mice 4 min after the 6 min forced swimming period was analyzed using the Forced Swim Scan™ 2.0 software.

The test results are expressed as mean ± SD. Comparisons between each group and the vehicle group were conducted using the t-test. The differences were considered statistically significant when $P < 0.05$.

18.2. Test results

Duloxetine, *N*-methyl-3-phenyl-3-[(benzo[*d*][1,3]dioxolan-4-yl)oxy]propylamine hydrochloride, (*S*)-*N*-methyl-3-phenyl-3-[(benzo[*d*][1,3]dioxolan-4-yl)oxy]propylamine hydrochloride, and the compound of formula (I) all show a relatively good dose-response relationship in each dose. The minimum effective dose of duloxetine is 5 mg/kg, and the $ED_{50}$ is 6.93 mg/kg. The minimum effective dose (MED) of *N*-methyl-3-phenyl-3-[(benzo[*d*][1,3]dioxolan-4-yl)oxy] propylamine hydrochloride is 0.4 mg/kg, and the median effective dose ($ED_{50}$) is 1.81 mg/kg; the MED of formula (*S*)-*N*-methyl-3-phenyl-3-[(benzo[*d*][1,3]dioxolan-4-yl)oxy]propylamine hydrochloride is 1.6 mg/kg, and the $ED_{50}$ is 1.74 mg/kg; the minimum effective dose of the compound of formula (I) is 0.4 mg/kg, and the $ED_{50}$ is 0.96 mg/kg. The specific results are shown in Table 11 below.

Table 11

| | n | Dose (mg/kg) | Immobility time (s) | $ED_{50}$ (mg/kg) |
|---|---|---|---|---|
| Vehicle | 12 | - | 172.5±43.7 | / |
| Duloxetine hydrochloride | 12 | 1.25 | 135.1±49.2 | 6.93 |
| | 12 | 2.5 | 154.9±76.0 | |
| | 12 | 5 | **104.9±51.5**** | |
| | 12 | 20 | **29.9±45.4**** | |
| | 12 | 40 | **10.6±17.3**** | |
| *N*-methyl-3-phenyl-3-[(benzo[*d*][1,3]dioxolan-4-yl)oxy]propylamine hydrochloride | 12 | 0.2 | 165.3±37.2 | 1.81 |
| | 12 | 0.4 | 125.6±53.6* | |
| | 12 | 0.8 | **86.9±53.4**** | |
| | 12 | 1.6 | **83.2±52.9**** | |
| | 12 | 15 | **64.3±73.7**** | |
| | 12 | 30 | **3.7±5.9**** | |
| (*S*)-N-methyl-3-phenyl-3-[(benzo[*d*][1,3]dioxolan-4-yl)oxy]propylamine hydrochloride | 12 | 0.2 | 180.4±29.4 | 1.74 |
| | 12 | 0.4 | 167.5±45.7 | |
| | 12 | 0.8 | 147.0±65.4 | |
| | 12 | 1.6 | **94.1±35.4**** | |
| | 12 | 15 | **8.9±8.7**** | |
| | 12 | 30 | **6.2±9.7**** | |

(continued)

| | n | Dose (mg/kg) | Immobility time (s) | $ED_{50}$ (mg/kg) |
|---|---|---|---|---|
| Compound of formula I | 12 | 0.2 | 178.6±17.0 | 0.96 |
| | 12 | 0.4 | **105.0±72.1*** | |
| | 12 | 0.8 | **106.0±59.7**** | |
| | 12 | 1.6 | **55.4±41.1**** | |
| | 12 | 15 | **13.0±23.3** | |
| | 12 | 30 | **7.6±4.4**** | |
| Note: *P < 0.05 and **P < 0.01 as compared with the vehicle group. | | | | |

18.3. Experimental conclusion: From the minimum effective dose, *N*-methyl-3-phenyl-3-[(benzo[*d*][1,3]dioxolan-4-yl)oxy]propylamine hydrochloride is the same as the compound of formula (I) (0.4 mg/kg), less than (*S*)-*N*-methyl-3-phenyl-3-[(benzo[*d*][1,3]dioxolan-4-yl)oxy]propylamine hydrochloride (1.6 mg/kg). From $ED_{50}$, the compound of formula (I) < (*S*)-*N*-methyl-3-phenyl-3-[(benzo[*d*][1,3]dioxolan-4-yl)oxy]propylamine hydrochloride < *N*-methyl-3-phenyl-3-[(benzo[*d*][1,3]dioxolan-4-yl)oxy]propylamine hydrochloride. The compound of formula (I) has a relatively good drug effect in mouse forced swimming by comprehensive evaluation.

**Example 19: Mouse acute toxicity test:**

19.1. Test method

The dose administered at the time of the formal test was determined based on the dose of the compound in the preliminary experiment that caused 100% and 0% death in mice. In the formal test, ICR mice were taken and randomly grouped, with 10 mice in each group, half male and half female, which were subjected to intragastric administration at 10 mL/kg. The animals were observed for state and mortality within 7 days.

19.2. Test results

The median lethal dose ($LD_{50}$) of *N*-methyl-3-phenyl-3-[(benzo[*d*][1,3]dioxolan-4-yl)oxy]propylamine hydrochloride in a single intragastric administration in mice is 112.3 mg/kg, and the 95% confidence interval is 102.1-121.9 mg/kg; the $LD_{50}$ of (*S*)-*N*-methyl-3-phenyl-3-[(benzo[*d*][1,3]dioxolan-4-yl)oxy]propylamine hydrochloride is 66.8 mg/kg, and the 95% confidence interval is 13.1-91.3 mg/kg; the $LD_{50}$ of the compound of formula (I) is 115.6 mg/kg, and the 95% confidence interval is 106.5-125.0 mg/kg.

19.3. Test conclusion

From the results of the mouse acute toxicity test, the compound of formula (I) is least toxic.

The therapeutic index (TI = $LD_{50}$ / $ED_{50}$) was calculated based on the results of the mouse acute toxicity and efficacy test. The results show that the therapeutic index of the compound of formula (I) is the largest. The specific data are shown in Table 12 below:

Table 12

| Serial number | Comparison content | (*S*)-*N*-methyl-3-phenyl-3- [(benzo [*d*][1,3]dioxolan-4-yl)oxy] propylamine hydrochloride | Compound of formula I | *N*-methyl-3-phenyl-3-[(benzo[*d*] [1,3]dioxolan-4-yl)oxy] propylamine hydrochloride |
|---|---|---|---|---|
| 1 | FST-$ED_{50}$ (mg/kg) | 1.74 | 0.96 | 1.81 |
| 2 | Mouse acute toxicity $LD_{50}$ (mg/kg) | 66.8 | 115.6 | 112.3 |
| 3 | TI-$LD_{50}$/$ED_{50}$ | 38.4 | 120.4 | 62.0 |

Summary: As can be seen from Example 1 to Example 19 and Table 1 to Table 12, *N*-methyl-3-phenyl-3-[(benzo[*d*][1,3]dioxolan-4-yl)oxy]propylamine hydrochloride, the compound of formula (I), and the A crystal form of the compound of formula (I) all have a relatively good solubility, minimum effective amount, and a relatively low toxicity. Moreover, the bioavailability of the compound of formula (I) and the A crystal form of the compound of formula (I) are both superior to their oxalates, and the A crystal form of the compound of formula (I) is still relatively stable under high temperature, high humidity, and 5000 lx illumination.

The above descriptions are only preferred examples of the present application and are not intended to limit the present invention, and various modifications and changes may be made by those skilled in the art. Any modifications, equivalent replacements, improvements, and the like that are made within the spirit and principle of the present application shall all fall within the protection scope of the present application.

## Claims

1. A hydrochloride of*N*-methyl-3-phenyl-3-[(benzo[*d*][1,3]dioxolan-4-yl)oxy]propylamine.

2. A hydrochloride of (*R*)-*N*-methyl-3-phenyl-3-[(benzo[*d*][1,3]dioxolan-4-yl)oxy]propylamine.

3. An A crystal form of the (R)-N-methyl-3-phenyl-3-[(benzo[*d*][1,3]dioxolan-4-yl)oxy]propylamine hydrochloride according to claim 2, wherein an X-ray powder diffraction pattern at 2θ angles of diffraction is obtained using Cu-Kα radiation, and the A crystal form shows characteristic peaks at 20 angles at 8.77°, 12.00°, and 14.84°, wherein the 2θ angle of each of the characteristic peaks has an error range of ±0.2°.

4. The A crystal form according to claim 3, wherein the A crystal form shows characteristic peaks at 2θ angles at 8.77°, 12.00°, 14.84°, 21.34°, and 21.87°, wherein the 2θ angle of each of the characteristic peaks has an error range of ±0.2°.

5. The A crystal form according to claim 3, wherein the A crystal form shows characteristic peaks at 20 angles at 8.77°, 12.00°, 14.84°, 21.34°, 21.87°, 23.47°, and 24.14°, wherein the 2θ angle of each of the characteristic peaks has an error range of ±0.2°.

6. The A crystal form according to any one of claims 3 to 5, wherein the X-ray powder diffraction pattern of the A crystal form further shows characteristic peaks at 2θ angles at one or more of 7.78°, 16.58°, 19.66°, 23.03°, 25.46°, 26.65°, 26.97°, 27.41°, and 28.23°, wherein the 2θ angle of each of the characteristic peaks has an error range of ±0.2°; preferably, the A crystal form has a X-ray powder diffraction pattern substantially consistent with FIG. 1.

7. The A crystal form according to claim 6, wherein the Raman spectrum of the A crystal form shows a characteristic peak at one or more of 3063.8±2 $cm^{-1}$, 3013.2±2 $cm^{-1}$, 2961.9±2 $cm^{-1}$, 2931.4±2 $cm^{-1}$, 1599.5±2 $cm^{-1}$, 1461.0±2 $cm^{-1}$, 1267.6±2 $cm^{-1}$, 1177.9±2 $cm^{-1}$, 1047.8±2 $cm^{-1}$, 996.6±2 $cm^{-1}$, 827.9±2 $cm^{-1}$, 740.3±2 $cm^{-1}$, 688.9±2 $cm^{-1}$, 615.4 ±2 $cm^{-1}$, and 591.9±2 $cm^{-1}$.

8. The A crystal form according to claim 7, wherein the melting endothermic peak of the DSC of the A crystal form is selected from 161.4 °C to 168.5 °C, preferably 164.8 °C.

9. A method for preparing the (*R*)-*N*-methyl-3-phenyl-3-[(benzo[d][1,3]dioxolan-4-yl)oxy]propylamine hydrochloride according to claim 2, comprising the following reaction steps:
after (*R*)-3-(dimethylamino)-1-phenylpropanol mandelate is freed, reacting with 3-fluoro-1,2-methylenedioxybenzene, and then performing demethylation to give (*R*)-*N*-methyl-3-phenyl-3-[(benzo[*d*][1,3]-dioxolan-4-yl)oxy]propylamine, which is formed into a salt using a HCl-ethyl acetate solution.

10. A method for preparing the A crystal form according to any one of claims 3-8, comprising the following steps:

    1) dissolving (*R*)-*N*-methyl-3-phenyl-3-[(benzo[*d*][1,3]dioxolan-4-yl)oxy]propylamine hydrochloride in a solvent to give a solution containing (*R*)-*N*-methyl-3-phenyl-3-[(benzo[*d*][1,3]dioxolan-4-yl)oxy]propylamine hydrochloride;
    2) removing the solvent in the solution prepared in step 1) by a solvent removal method to give a precipitate;

    wherein the solvent in step 1) is selected from a good solvent or a mixed solvent of a good solvent and a poor solvent,

wherein the good solvent is one or more of alcohols, halogenated hydrocarbons, *N*-methyl-2-pyrrolidone, nitriles, water, *N*,*N*-dimethylformamide, or dimethyl sulfoxide; the alcohol is preferably selected from methanol, ethanol, *n*-propanol, isopropanol, or n-butanol, the halogenated hydrocarbon is preferably selected from dichloromethane or chloroform, and the nitrile is preferably selected from acetonitrile; the poor solvent is selected from $C_{5-10}$ saturated hydrocarbons, ketones, esters, or ethers; the $C_{5-10}$ saturated hydrocarbon is preferably selected from *n*-pentane, *n*-hexane, cyclohexane, or *n*-heptane, the ketone is preferably selected from acetone and butanone, the ester is preferably selected from ethyl acetate, n-propyl acetate, isopropyl acetate, or isobutyl acetate, and the ether is preferably selected from methyl *tert*-butyl ether, isopropyl ether, petroleum ether, or tetrahydrofuran.

**11.** A method for preparing the A crystal form according to any one of claims 3-8, comprising the following steps:

1) dissolving formula (*R*)-*N*-methyl-3-phenyl-3-[(benzo[*d*][1,3]dioxolan-4-yl)oxy]propylamine hydrochloride in a solvent to give a solution containing (*R*)-*N*-methyl-3-phenyl-3-[(benzo[*d*][1,3]dioxolan-4-yl)oxy]propylamine hydrochloride;
2) obtaining a precipitate from the solution obtained in step 1) by a precipitation method,
wherein the solvent in step 1) is selected from a good solvent or a mixed solvent of a good solvent and a poor solvent, wherein the good solvent is one or more of alcohols, halogenated hydrocarbons, *N*-methyl-2-pyrrolidone, nitriles, water, *N*,*N*-dimethylformamide, or dimethyl sulfoxide; the alcohol is preferably selected from methanol, ethanol, *n*-propanol, isopropanol, or n-butanol, the halogenated hydrocarbon is preferably selected from dichloromethane or chloroform, and the nitrile is preferably selected from acetonitrile; the poor solvent is selected from $C_{5-10}$ saturated hydrocarbons, ketones, esters, or ethers; the $C_{5-10}$ saturated hydrocarbon is preferably selected from *n*-pentane, *n*-hexane, cyclohexane, or *n*-heptane, the ketone is preferably selected from acetone and butanone, the ester is preferably selected from ethyl acetate, *n*-propyl acetate, isopropyl acetate, or isobutyl acetate, and the ether is preferably selected from methyl *tert*-butyl ether, isopropyl ether, petroleum ether, or tetrahydrofuran;
the precipitation method is selected from a cooling method or a precipitant method;
the cooling method is cooling the solution obtained in step 1) to 0 °C-room temperature so that a crystal is precipitated;
the precipitant method is adding a precipitant of (*R*)-*N*-methyl-3-phenyl-3-[(benzo[*d*][1,3]dioxolan-4-yl)oxy]propylamine hydrochloride to the solution obtained in step 1) so that a crystal is precipitated.

**12.** The method for preparing the A crystal form according to claim 11, wherein the precipitant is selected from $C_{5-10}$ saturated hydrocarbons, ketones, esters, or ethers; the $C_{5-10}$ saturated hydrocarbon is preferably selected from *n*-pentane, *n*-hexane, cyclohexane, or *n*-heptane, the ketone is preferably selected from acetone and butanone, the ester is preferably selected from ethyl acetate, *n*-propyl acetate, isopropyl acetate, or isobutyl acetate, and the ether is preferably selected from methyl *tert*-butyl ether, isopropyl ether, petroleum ether, or tetrahydrofuran.

**13.** A pharmaceutical composition comprising the hydrochloride according to claim 1 or the hydrochloride according to claim 2 or the A crystal form according to any one of claims 3-8, and one or more excipients, carriers, adjuvants or vehicles or a combination thereof.

**14.** Use of the hydrochloride according to claim 1 or 2, or the A crystal form according to any one of claims 3-8, or the pharmaceutical composition according to claim 13 for preparing a medicament for treating and/or preventing a psychiatric disease, wherein the psychiatric disease is selected from one or more of anxiety, obsessive-compulsive disorder, depression, phobia, and schizophrenia, preferably depression.

**15.** A method for treating and/or preventing a psychiatric disease comprising administering to a patient the hydrochloride according to claim 1 or 2 or the A crystal form according to any one of claims 3-8 or the pharmaceutical composition according to claim 13,
wherein the psychiatric disease is selected from one or more of anxiety, obsessive-compulsive disorder, depression, phobia, and schizophrenia, preferably depression.

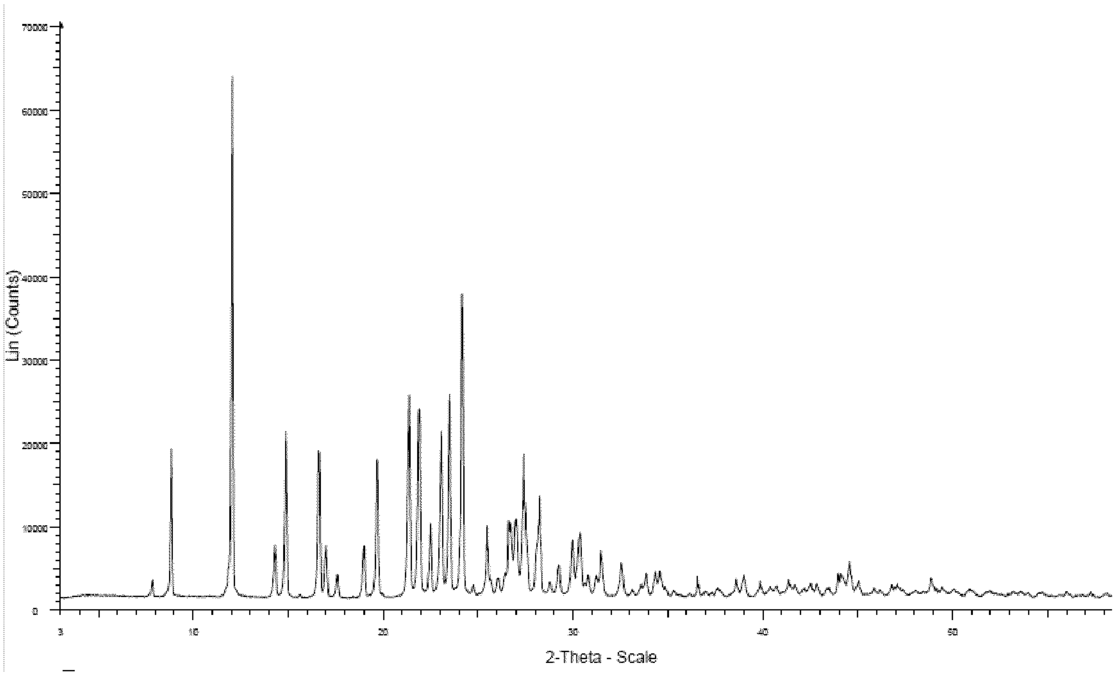
70000
60000
50000
40000
30000
20000
10000
0
Lin (Counts)
3
10
20
30
40
50
2-Theta - Scale

FIG. 1

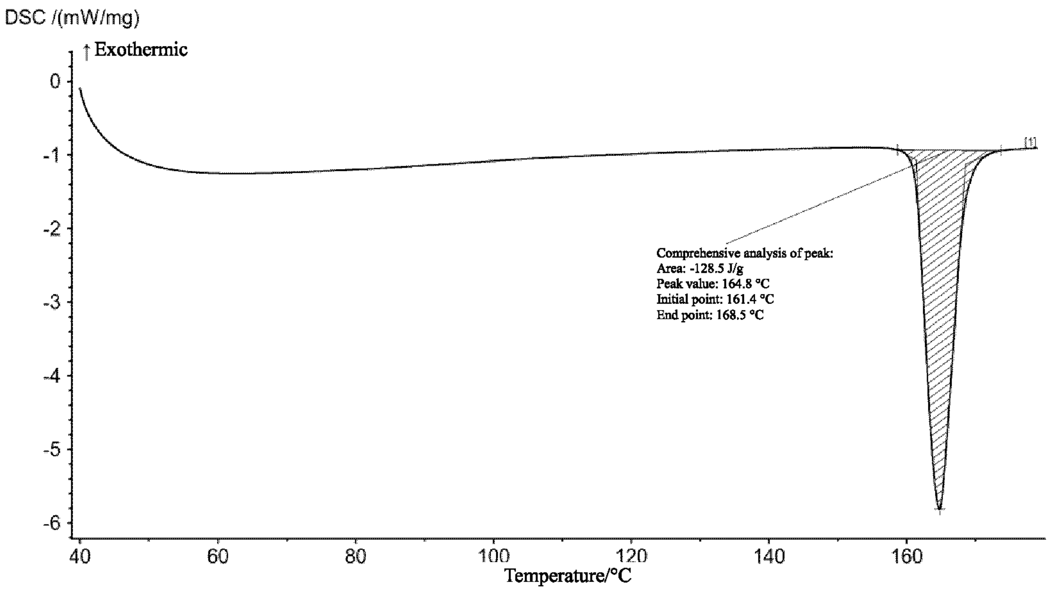
DSC /(mW/mg)
↑ Exothermic
0
-1
-2
-3
-4
-5
-6
[1]
Comprehensive analysis of peak:
Area: -128.5 J/g
Peak value: 164.8 °C
Initial point: 161.4 °C
End point: 168.5 °C
40
60
80
100
120
140
160
Temperature/°C

FIG. 2

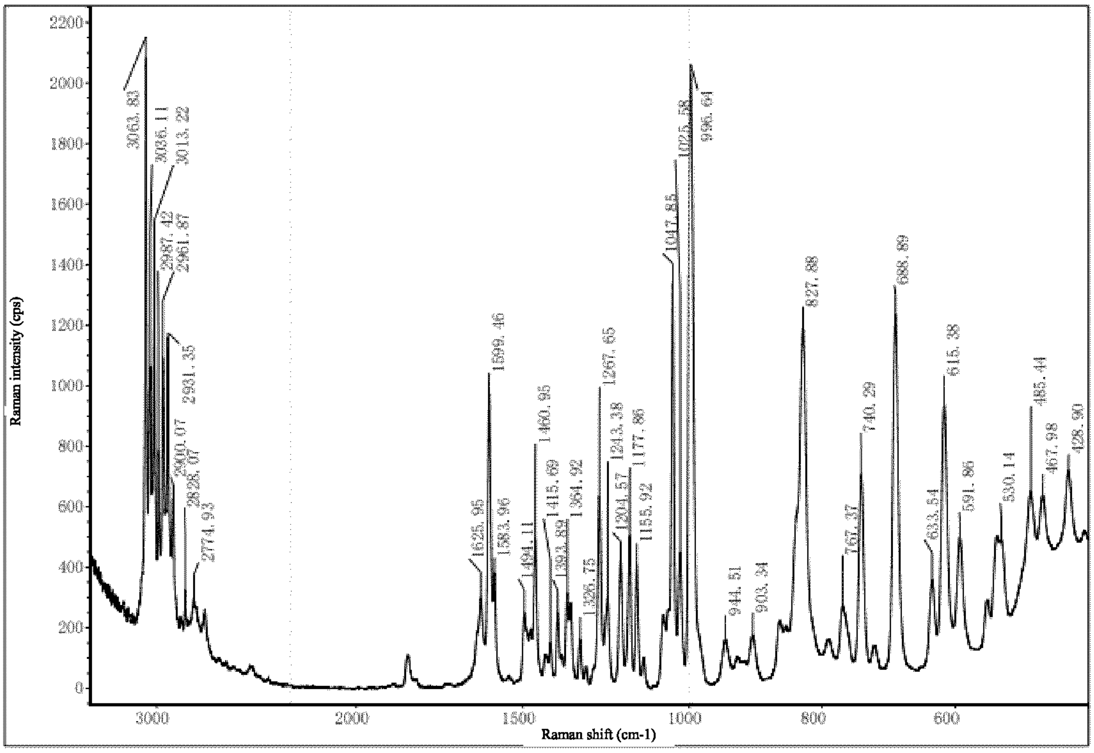

Raman intensity (cps)
Raman shift (cm-1)
3063. 83
3036. 11
3013. 22
2987. 42
2961. 87
2931. 35
2900. 07
2828. 07
2774. 93
1625. 95
1599. 46
1583. 96
1494. 11
1460. 95
1415. 69
1393. 89
1364. 92
1326. 75
1267. 65
1243. 38
1204. 57
1177. 86
1155. 92
1047. 85
1025. 58
996. 64
944. 51
903. 34
827. 88
767. 37
740. 29
688. 89
633. 54
615. 38
591. 86
530. 14
485. 44
467. 98
428. 90


FIG. 3

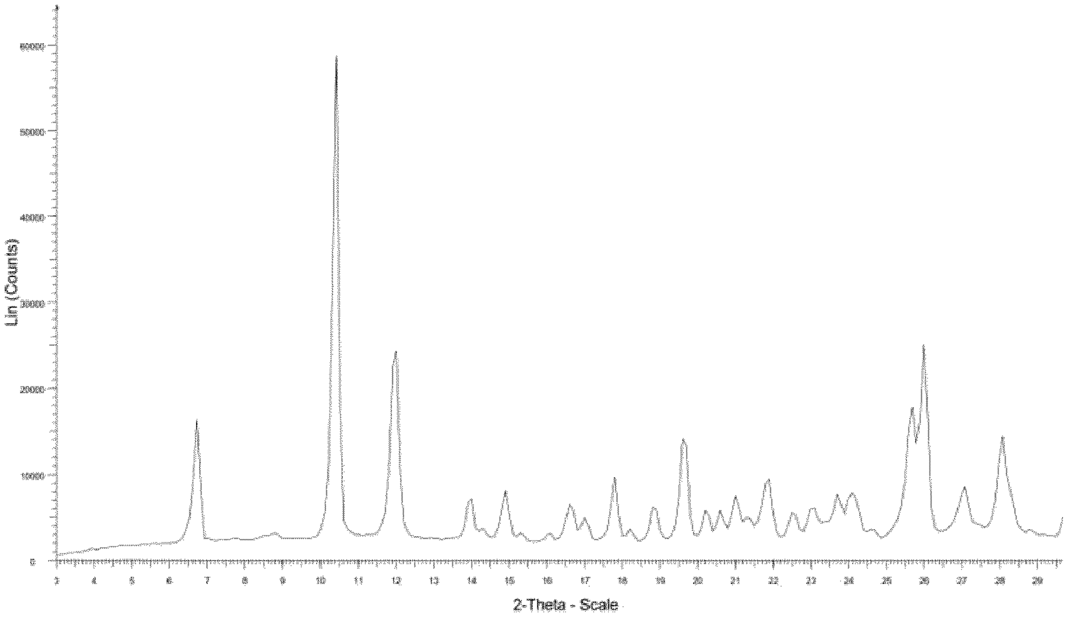

Lin (Counts)
2-Theta - Scale


FIG. 4

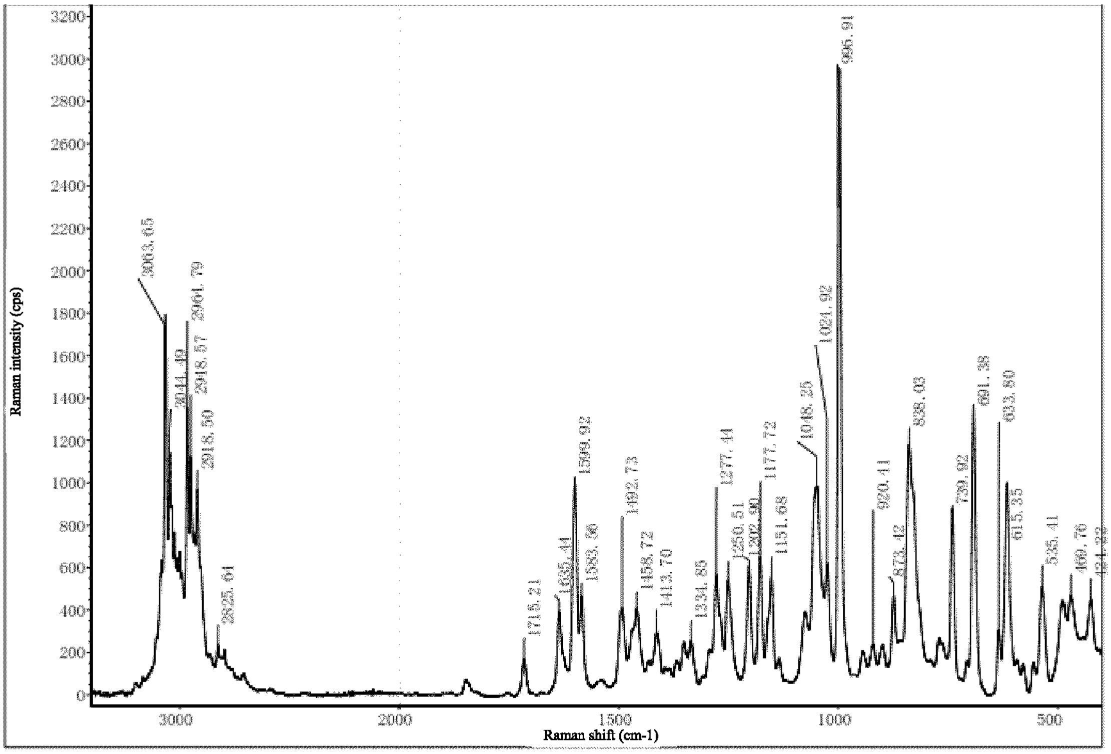
Raman intensity (cps)
Raman shift (cm-1)
0
200
400
600
800
1000
1200
1400
1600
1800
2000
2200
2400
2600
2800
3000
3200
3000
2000
1500
1000
500
3063.65
3044.49
2964.79
2948.57
2918.50
2825.64
1715.21
1635.44
1599.92
1583.56
1492.73
1458.72
1413.70
1334.85
1277.44
1250.51
1202.90
1177.72
1151.68
1048.25
1021.92
996.91
920.41
873.42
838.03
739.92
691.38
633.80
615.35
535.41
469.76

FIG. 5

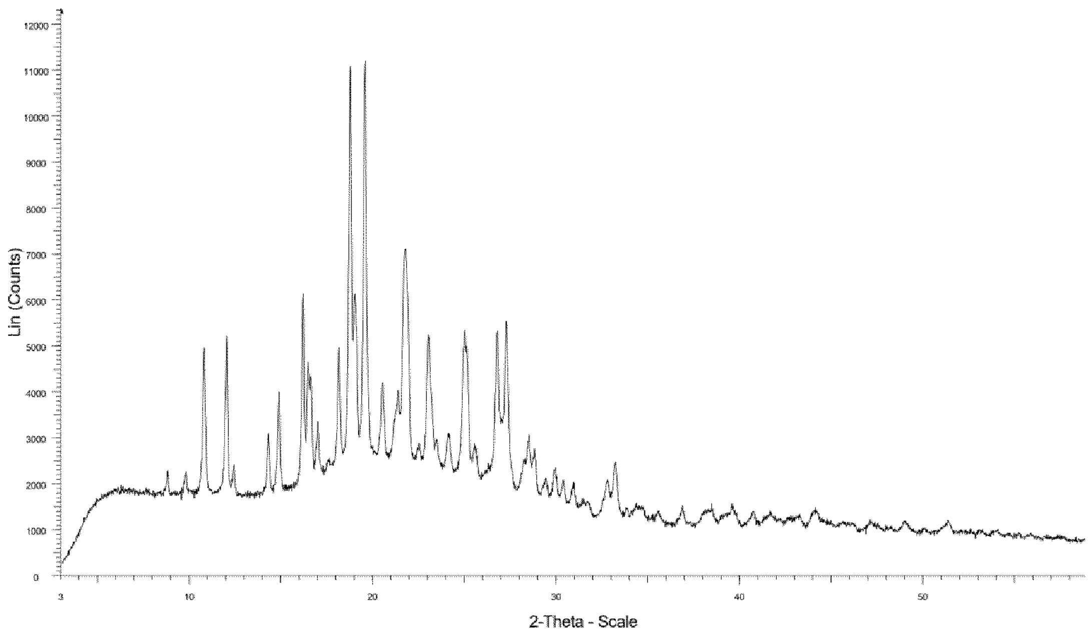
Lin (Counts)
2-Theta - Scale
0
1000
2000
3000
4000
5000
6000
7000
8000
9000
10000
11000
12000
3
10
20
30
40
50

FIG. 6

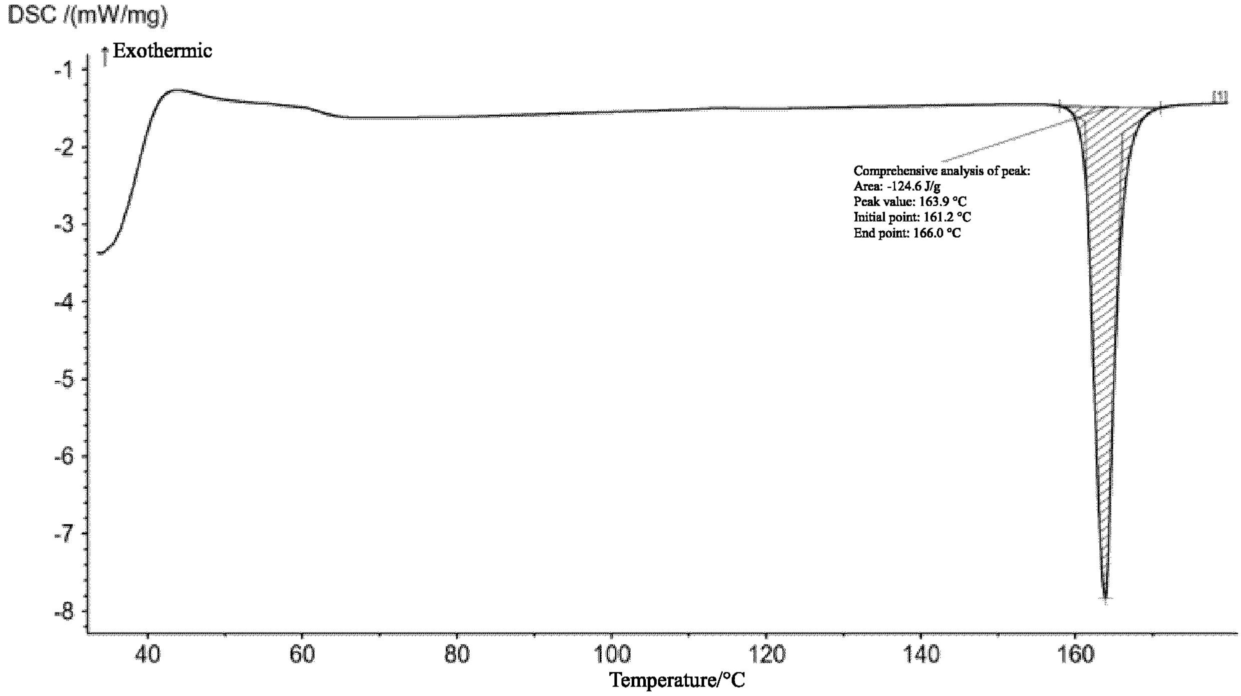
DSC /(mW/mg)
↑Exothermic
-1
-2
-3
-4
-5
-6
-7
-8
40
60
80
100
120
140
160
Temperature/°C
[1]
Comprehensive analysis of peak:
Area: -124.6 J/g
Peak value: 163.9 °C
Initial point: 161.2 °C
End point: 166.0 °C

FIG. 7

**INTERNATIONAL SEARCH REPORT**

International application No.
**PCT/CN2023/073367**

**A. CLASSIFICATION OF SUBJECT MATTER**

C07D317/64(2006.01)i;A61K31/36(2006.01)i;A61P25/24(2006.01)i

According to International Patent Classification (IPC) or to both national classification and IPC

**B. FIELDS SEARCHED**

Minimum documentation searched (classification system followed by classification symbols)

IPC: C07D317/-, A61K31/-, A61P25/-

Documentation searched other than minimum documentation to the extent that such documents are included in the fields searched

Electronic data base consulted during the international search (name of data base and, where practicable, search terms used)

CNABS, CNTXT, VEN, WPI, CNKI, STN (CAPLUS, REGISTRY): 1955463-43-0/RN, 1955463-42-9/RN, 式(I)结构式, formula (I), 盐, 晶型, 抑郁症, salt, crystal?, antidepressant

**C. DOCUMENTS CONSIDERED TO BE RELEVANT**

| Category* | Citation of document, with indication, where appropriate, of the relevant passages | Relevant to claim No. |
|---|---|---|
| A | WO 2016101898 A1 (NHWA PHARMA CORP.) 30 June 2016 (2016-06-30)<br>entire document, in particular claims 1-10 and embodiment 4, compound 2 | 1-15 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 April 2023** | **13 April 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer |
| Facsimile No. **(86-10)62019451** | Telephone No. |

Form PCT/ISA/210 (second sheet) (July 2022)

**INTERNATIONAL SEARCH REPORT**

International application No.
**PCT/CN2023/073367**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **15**
because they relate to subject matter not required to be searched by this Authority, namely:

Although claim 15 relates to a method for prevention and/or treatment of diseases which is implemented on the human body (PCT Rules 39.1(iv) and 67.1(iv)), a search is carried out in the search report according to the activity of a compound claimed in the application document.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/073367**

| Patent document cited in search report | Publication date (day/month/year) | Patent family member(s) | Publication date (day/month/year) |
|---|---|---|---|
| WO 2016101898 A1 | 30 June 2016 | US 2017369466 A1 | 28 December 2017 |
| | | US 10093644 B2 | 09 October 2018 |
| | | JP 2018501316 A | 18 January 2018 |
| | | JP 6426302 B2 | 21 November 2018 |
| | | CN 105777706 A | 20 July 2016 |
| | | CN 105777706 B | 23 August 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016101898 A
- CN 105777706 A


### Non-patent literature cited in the description


- **JACQUES et al.** Enantiomers, Racemates and Resolutions. Wiley Interscience, 1981
- **WILEN et al.** *Tetrahedron*, 1977, vol. 33, 2725
- **ELIEL**. Stereochemistry of Carbon Compounds. McGraw-Hill, 1962
- **WILEN**. Tables of Resolving Agents and Optical Resolutions. Univ. of Notre Dame Press, 1972, 268
- **LIEBERMAN**. Pharmaceutical Dosage Forms. 1992, vol. 1-3
- **LLOYD**. The Art, Science and Technology of Pharmaceutical Compounding. 1999
- **PICKAR**. *Dosage Calculations*, 1999
- Remington: The Science and Practice of Pharmacy. Lippincott, Williams & Wilkins, 2003